# EUROPEAN PATENT APPLICATION

(11) **EP 4 484 445 A1**
(43) Date of publication of application: **01.01.2025**
(21) Application number: 23181563.0
(22) Date of filing: 26.06.2023
(51) Int. Cl.: C07K 16/08

(54) **HCMV NEUTRALIZING ANTIBODIES**

(71) Applicant: Universität zu Köln, 50923 Köln (DE); Universität Ulm, 89081 Ulm (DE); Universität Duisburg-Essen, 45141 Essen (DE)
(72) Inventor: KLEIN, Florian, 50933 Köln (DE); SINZGER, Christian, 89081 Ulm (DE); KRAWCZYK, Adalbert, 45257 Essen (DE); ALT, Mira, 45147 Essen (DE); ZEHNER, Matthias, 53332 Bornheim (DE)
(74) Representative: Klöckner, Christoph

(57) **Abstract**

The present invention relates to antibodies or antigen-binding fragments thereof against Human Cytomegalovirus (HCMV), pharmaceutical compositions comprising such antibodies or antigen-binding fragments thereof, kits comprising such antibodies or antigen-binding fragments thereof, as well as the antibodies or antigen-binding fragments thereof, the pharmaceutical compositions, and the kits for use as a medicament, and in the treatment or prevention of an infection with Human Cytomegalovirus. The present invention further relates to nucleic acids encoding the aforementioned such antibodies or antigen-binding fragments thereof, expression vectors comprising such nucleic acids, host cells comprising such nucleic acids or expression vectors, and methods for the production of such antibodies or antigen-binding fragments thereof.

## Description

### Technical Field

The present invention relates to antibodies or antigen-binding fragments thereof against Human Cytomegalovirus (HCMV), pharmaceutical compositions comprising such antibodies or antigen-binding fragments thereof, kits comprising such antibodies or antigen-binding fragments thereof, and the antibodies or antigen-binding fragments thereof, the pharmaceutical compositions and the kits for use as a medicament, and in the treatment or prevention of an infection with Human Cytomegalovirus. The present invention further relates to nucleic acids encoding such antibodies or antigen-binding fragments thereof, expression vectors comprising such nucleic acids, host cells comprising such nucleic acids or expression vectors, and methods for the production of such antibodies or antigen-binding fragments thereof.

### Technological Background

Human cytomegalovirus (HCMV) is a ubiquitous virus that occurs worldwide and can cause lifelong latency in humans. The infection is typically asymptomatic but can lead to severe disease in immunocompromised patients and is a major cause of congenital birth defects. Nevertheless, there is currently no approved vaccine against HCMV and antiviral drugs are often limited by inadequate efficacy, emergence of drug-resistant strains, and associated side effects (El Helou and Razonable, 2019). Therefore, new approaches are urgently needed to prevent and treat HCMV infections.

HCMV uses a complex machinery of multiple proteins for the infection of different target cells. After initial attachment, the Trimer complex (gH/gL/gO) and the Pentamer complex (gH/gL/UL_{128/130/131A}) are essential for viral entry into host cells and have been shown to bind to cellular receptors, including PDGFRα (platelet-derived growth factor receptor alpha) and TGFBR3 (transforming growth factor beta receptor 3) for the Trimer, as well as NRP2 (neuropilin-2) and THBD (thrombomodulin) for the Pentamer.

The Trimer is required for viral entry into most target cells such as epithelial and endothelial cells, and fibroblasts. The Pentamer induces endocytosis of HCMV particles and determines viral tropism for epithelial- and endothelial cells, as well as leukocytes.

Recent data indicate that antibody (Ab)-mediated immunity contributes to the control of HCMV infection. Thus, antibodies are considered as a promising option for the prevention and treatment of HCMV infections (Sandonís et al., 2020). Previous quantitative analyses indicated the Trimer and the Pentamer, together with fusion protein gB, as major antibody targets on the HCMV surface.

Some neutralizing antibodies (nAbs) targeting gB or gH have been reported to diminish HCMV infections of the majority of target cells, but the neutralization potency of these nAbs was moderate. In contrast, nAbs directed against UL_{128/130/131A}-subunits of the Pentamer were demonstrated to potently neutralize the infection of epithelial- and endothelial cells, but failed to suppress HCMV fibroblast infection (Gerna et al., 2016).

Moreover, it was recently shown that the gO subunit of the Trimer is essential for infection of all cell types and is discussed to play an important role in viral assembly (Zhou, Lanchy, and Ryckman, 2015). Nevertheless, only one gO-specific nAb has been described to date.

Clinical trials exploring the performance of gB- and Pentamer-interacting antibodies were performed during the last years (e.g. MSL109 in context of CMV retinitis), but failed to protect from HCMV infection. Furthermore, the potency of antibody cocktails such as RG7667, a combination of a gH-(MCMV5322A) and a humanized UL_{128/130/131A}-specific antibody (MCMV3068A), was evaluated.

Although these cocktails did not achieve the anticipated levels of activity, HCMV viremia was reduced and delayed in RG7667-treated patients. Moreover, the incidence of CMV disease was reduced, highlighting the potential of HCMV-antibody treatment (Ishida et al., 2017). However, in order to fully utilize the capabilities of antibodies and derive benefits in the battle against HCMV, improved antibody-mediated HCMV neutralization is required.

Thus, it is an object of the present invention to provide novel monoclonal antibodies against HCMV which demonstrate superior neutralizing activity.

It is a further object of the present invention to provide novel monoclonal antibodies directed against novel epitopes of the HCMV virus within the trimeric and pentameric complexes including the gO subunit and the gH/gL subunit. It is also an object of the present invention to provide novel monoclonal antibodies which are able to neutralize infection of epithelial cells and endothelial cells and to suppress fibroblast infection.

### Summary of the invention

These objects have been solved by the present invention as specified hereinafter.

According to a first aspect of the present invention, an antibody or antigen-binding fragment thereof directed against *Human Cytomegalovirus (HCMV; Human Betaherpesvirus 5)* is provided, wherein the antibody or antigen-binding fragment thereof comprises the combination of the heavy chain CDR1 to CDR3 and the light chain CDR1 to CDR3 amino acid sequence of one antibody selected from the group comprising **CS2it1p2_F7K** (having a CDR-H1 amino acid sequence of SEQ ID No. 1, a CDR-H2 amino acid sequence of SEQ ID No. 2, a CDR-H3 amino acid sequence of SEQ ID No. 3, a CDR-L1 amino acid sequence of SEQ ID No. 4, a CDR-L2 amino acid sequence of SEQ ID No. 5, a CDR-L3 amino acid sequence of SEQ ID No. 6), **CS4tt1p1_E3K** (having a CDR-H1 amino acid sequence of SEQ ID No. 7, a CDR-H2 amino acid sequence of SEQ ID No. 8, a CDR-H3 amino acid sequence of SEQ ID No. 9, a CDR-L1 amino acid sequence of SEQ ID No. 10, a CDR-L2 amino acid sequence of SEQ ID No. 11, a CDR-L3 amino acid sequence of SEQ ID No. 12), **CS1pt2p3_E1K** (having a CDR-H1 amino acid sequence of SEQ ID No. 13, a CDR-H2 amino acid sequence of SEQ ID No. 14, a CDR-H3 amino acid sequence of SEQ ID No. 15, a CDR-L1 amino acid sequence of SEQ ID No. 16, a CDR-L2 amino acid sequence of SEQ ID No. 17, a CDR-L3 amino acid sequence of SEQ ID No. 18), **CS2pt1p2_E12K** (having a CDR-H1 amino acid sequence of SEQ ID No. 19, a CDR-H2 amino acid sequence of SEQ ID No. 20, a CDR-H3 amino acid sequence of SEQ ID No. 21, a CDR-L1 amino acid sequence of SEQ ID No. 22, a CDR-L2 amino acid sequence of SEQ ID No. 23, a CDR-L3 amino acid sequence of SEQ ID No. 24), **CS2pt1p2_A10L** (having a CDR-H1 amino acid sequence of SEQ ID No. 25, a CDR-H2 amino acid sequence of SEQ ID No. 26, a CDR-H3 amino acid sequence of SEQ ID No. 27, a CDR-L1 amino acid sequence of SEQ ID No. 28, a CDR-L2 amino acid sequence of SEQ ID No. 29, a CDR-L3 amino acid sequence of SEQ ID No. 30), **CS2tt1p1_C11L** (having a CDR-H1 amino acid sequence of SEQ ID No. 31, a CDR-H2 amino acid sequence of SEQ ID No. 32, a CDR-H3 amino acid sequence of SEQ ID No. 33, a CDR-L1 amino acid sequence of SEQ ID No. 34, a CDR-L2 amino acid sequence of SEQ ID No. 35, a CDR-L3 amino acid sequence of SEQ ID No. 36), **CS3tt1p3_A12K** (having a CDR-H1 amino acid sequence of SEQ ID No. 37, a CDR-H2 amino acid sequence of SEQ ID No. 38, a CDR-H3 amino acid sequence of SEQ ID No. 39, a CDR-L1 amino acid sequence of SEQ ID No. 40, a CDR-L2 amino acid sequence of SEQ ID No. 41, a CDR-L3 amino acid sequence of SEQ ID No. 42), **CS3pt1p1_E4K** (having a CDR-H1 amino acid sequence of SEQ ID No. 43, a CDR-H2 amino acid sequence of SEQ ID No. 44, a CDR-H3 amino acid sequence of SEQ ID No. 45, a CDR-L1 amino acid sequence of SEQ ID No. 46, a CDR-L2 amino acid sequence of SEQ ID No. 47, a CDR-L3 amino acid sequence of SEQ ID No. 48), **CS2tt1p3_C3K** (having a CDR-H1 amino acid sequence of SEQ ID No. 49, a CDR-H2 amino acid sequence of SEQ ID No. 50, a CDR-H3 amino acid sequence of SEQ ID No. 51, a CDR-L1 amino acid sequence of SEQ ID No. 52, a CDR-L2 amino acid sequence of SEQ ID No. 53, a CDR-L3 amino acid sequence of SEQ ID No. 54), **CS2pt1p3_E12K** (having a CDR-H1 amino acid sequence of SEQ ID No. 55, a CDR-H2 amino acid sequence of SEQ ID No. 56, a CDR-H3 amino acid sequence of SEQ ID No. 57, a CDR-L1 amino acid sequence of SEQ ID No. 58, a CDR-L2 amino acid sequence of SEQ ID No. 59, a CDR-L3 amino acid sequence of SEQ ID No. 60), **CS3tt1p1_G9K** (having a CDR-H1 amino acid sequence of SEQ ID No. 61, a CDR-H2 amino acid sequence of SEQ ID No. 62, a CDR-H3 amino acid sequence of SEQ ID No. 63, a CDR-L1 amino acid sequence of SEQ ID No. 64, a CDR-L2 amino acid sequence of SEQ ID No. 65, a CDR-L3 amino acid sequence of SEQ ID No. 66), **CS3tt1p4_G2K** (having a CDR-H1 amino acid sequence of SEQ ID No. 67, a CDR-H2 amino acid sequence of SEQ ID No. 68, a CDR-H3 amino acid sequence of SEQ ID No. 69, a CDR-L1 amino acid sequence of SEQ ID No. 70, a CDR-L2 amino acid sequence of SEQ ID No. 71, a CDR-L3 amino acid sequence of SEQ ID No. 72), **CS3tt1p1_A9K** (having a CDR-H1 amino acid sequence of SEQ ID No. 73, a CDR-H2 amino acid sequence of SEQ ID No. 74, a CDR-H3 amino acid sequence of SEQ ID No. 75, a CDR-L1 amino acid sequence of SEQ ID No. 76, a CDR-L2 amino acid sequence of SEQ ID No. 77, a CDR-L3 amino acid sequence of SEQ ID No. 78), **CS2pt1p1_E1K** (having a CDR-H1 amino acid sequence of SEQ ID No. 79, a CDR-H2 amino acid sequence of SEQ ID No. 80, a CDR-H3 amino acid sequence of SEQ ID No. 81, a CDR-L1 amino acid sequence of SEQ ID No. 82, a CDR-L2 amino acid sequence of SEQ ID No. 83, a CDR-L3 amino acid sequence of SEQ ID No. 84), and **CS3pt1p4**_**C1L** (having a CDR-H1 amino acid sequence of SEQ ID No. 113, a CDR-H2 amino acid sequence of SEQ ID No. 114, a CDR-H3 amino acid sequence of SEQ ID No. 115, a CDR-L1 amino acid sequence of SEQ ID No. 116, a CDR-L2 amino acid sequence of SEQ ID No. 117, a CDR-L3 amino acid sequence of SEQ ID No. 118).

In one embodiment of the first aspect of the present invention, the antibody or antigen-binding fragment thereof comprises the combination of the variable region heavy chain amino acid sequence and of the variable region light chain amino acid sequence of one antibody selected from the group comprising **CS2it1p2_F7K** (having the variable region heavy chain amino acid sequence of SEQ ID No. 85 and the variable region light chain amino acid sequence of SEQ ID No. 86), **CS4tt1p1_E3K** (having the variable region heavy chain amino acid sequence of SEQ ID No. 87 and the variable region light chain amino acid sequence of SEQ ID No. 88), **CS1pt2p3_E1K** (having the variable region heavy chain amino acid sequence of SEQ ID No. 89 and the variable region light chain amino acid sequence of SEQ ID No. 90), **CS2pt1p2_E12K** (having the variable region heavy chain amino acid sequence of SEQ ID No. 91 and the variable region light chain amino acid sequence of SEQ ID No. 92), **CS2pt1p2_A10L** (having the variable region heavy chain amino acid sequence of SEQ ID No. 93 and the variable region light chain amino acid sequence of SEQ ID No. 94), **CS2tt1p1_C11L** (having the variable region heavy chain amino acid sequence of SEQ ID No. 95 and the variable region light chain amino acid sequence of SEQ ID No. 96), **CS3tt1p3_A12K** (having the variable region heavy chain amino acid sequence of SEQ ID No. 97 and the variable region light chain amino acid sequence of SEQ ID No. 98), **CS3pt1p1_E4K** (having the variable region heavy chain amino acid sequence of SEQ ID No. 99 and the variable region light chain amino acid sequence of SEQ ID No. 100), **CS2tt1p3_C3K** (having the variable region heavy chain amino acid sequence of SEQ ID No. 101 and the variable region light chain amino acid sequence of SEQ ID No. 102), **CS2pt1p3_E12K** (having the variable region heavy chain amino acid sequence of SEQ ID No. 103 and the variable region light chain amino acid sequence of SEQ ID No. 104), **CS3tt1p1_G9K** (having the variable region heavy chain amino acid sequence of SEQ ID No. 105 and the variable region light chain amino acid sequence of SEQ ID No. 106), **CS3tt1p4_G2K** (having the variable region heavy chain amino acid sequence of SEQ ID No. 107 and the variable region light chain amino acid sequence of SEQ ID No. 108), **CS3tt1p1_A9K** (having the variable region heavy chain amino acid sequence of SEQ ID No. 109 and the variable region light chain amino acid sequence of SEQ ID No. 110), **CS2pt1p1_E1K** (having the variable region heavy chain amino acid sequence of SEQ ID No. 111 and the variable region light chain amino acid sequence of SEQ ID No. 112), and **CS3pt1p4_C1L** (having the variable region heavy chain amino acid sequence of SEQ ID No. 119 and the variable region light chain amino acid sequence of SEQ ID No. 120).

In one embodiment of the first aspect of the invention, the amino acid sequences comprised are of one antibody selected from the group comprising CS2it1p2_F7K, CS4tt1p1_E3K, CS1pt2p3_E1K, CS2pt1p2_E12K, CS2pt1p2_A10L, CS2tt1p1_C11L, CS3tt1p3_A12K, and CS3pt1p1_E4K, preferably of one antibody from the group comprising CS2it1p2_F7K, CS4tt1p1_E3K, CS1pt2p3_E1K, CS2pt1p2_E12K, CS2pt1p2_A10L, and CS2tt1p1_C11L, more preferably of one antibody from the group comprising CS2it1p2_F7K, CS4tt1p1_E3K, CS1pt2p3_E1K, and CS2pt1p2_E12K, particularly preferably of the antibody CS2it1p2_F7K.

According to a second aspect of the present invention, an antibody or antigen-binding fragment thereof is provided that specifically binds to the gO glycoprotein of Human Cytomegalovirus.

In one embodiment of the second aspect of the invention, the antibody or antigen-binding fragment thereof competes with an antibody comprising the combination of the variable region heavy chain amino acid sequence of SEQ ID No. 85 and of the variable region light chain amino acid sequence of SEQ ID No. 86 of antibody CS2it1p2_F7K for binding to Human Cytomegalovirus.

According to a third aspect of the present invention, an antibody or antigen-binding fragment thereof is provided that competes with an antibody comprising the combination of the variable region heavy chain amino acid sequence of SEQ ID No. 87 and of the variable region light chain amino acid sequence of SEQ ID No. 88 of antibody CS4tt1p1_E3K for binding to Human Cytomegalovirus, or
an antibody or antigen-binding fragment thereof that competes with an antibody comprising the combination of the variable region heavy chain amino acid sequence of SEQ ID No. 89 and of the variable region light chain amino acid sequence of SEQ ID No. 90 of antibody CS1pt2p3_E1K for binding to Human Cytomegalovirus, or
an antibody or antigen-binding fragment thereof that competes with an antibody comprising the combination of the variable region heavy chain amino acid sequence of SEQ ID No. 91 and of the variable region light chain amino acid sequence of SEQ ID No. 92 of antibody CS2pt1p2_E12K for binding to Human Cytomegalovirus, or
an antibody or antigen-binding fragment thereof that competes with an antibody comprising the combination of the variable region heavy chain amino acid sequence of SEQ ID No. 93 and of the variable region light chain amino acid sequence of SEQ ID No. 94 of antibody CS2pt1p2_A10L for binding to Human Cytomegalovirus, or
an antibody or antigen-binding fragment thereof that competes with an antibody comprising the combination of the variable region heavy chain amino acid sequence of SEQ ID No. 97 and of the variable region light chain amino acid sequence of SEQ ID No. 98 of antibody CS3tt1p3_A12K for binding to Human Cytomegalovirus, or
an antibody or antigen-binding fragment thereof that competes with an antibody comprising the combination of the variable region heavy chain amino acid sequence of SEQ ID No. 99 and of the variable region light chain amino acid sequence of SEQ ID No. 100 of antibody CS3pt1p1_E4K for binding to Human Cytomegalovirus,
or an antibody or antigen-binding fragment thereof that competes with an antibody comprising the combination of the variable region heavy chain amino acid sequence of SEQ ID No. 101 and of the variable region light chain amino acid sequence of SEQ ID No. 102 of antibody CS2tt1p3_C3K for binding to Human Cytomegalovirus,
or an antibody or antigen-binding fragment thereof that competes with an antibody comprising the combination of the variable region heavy chain amino acid sequence of SEQ ID No. 103 and of the variable region light chain amino acid sequence of SEQ ID No. 104 of antibody **CS2pt1**p3_E12K for binding to Human Cytomegalovirus, or
an antibody or antigen-binding fragment thereof that competes with an antibody comprising the combination of the variable region heavy chain amino acid sequence of SEQ ID No. 105 and of the variable region light chain amino acid sequence of SEQ ID No. 106 of antibody CS3tt1p1_G9K for binding to Human Cytomegalovirus, or
an antibody or antigen-binding fragment thereof that competes with an antibody comprising the combination of the variable region heavy chain amino acid sequence of SEQ ID No. 109 and of the variable region light chain amino acid sequence of SEQ ID No. 110 of antibody CS3tt1p1_A9K for binding to Human Cytomegalovirus, or
an antibody or antigen-binding fragment thereof that competes with an antibody comprising the combination of the variable region heavy chain amino acid sequence of SEQ ID No. 111 and of the variable region light chain amino acid sequence of SEQ ID No. 112 of antibody CS2pt1p1_E1K for binding to Human Cytomegalovirus.

In one embodiment of the first, second or third aspects of the present invention, the amino acid sequences of the CDRs or of the variable regions comprised therein are from an antibody which is able to neutralize Human Cytomegalovirus at a lower IC50 than reference antibody MCMV5322A (as described in Deng et al., 2018).

In another embodiment of the first, second or third aspects of the present invention, the amino acid sequences of the CDRs or of the variable regions comprised therein are from an antibody which is able to neutralize Human Cytomegalovirus at a lower IC50 than reference antibody 4I22 (as described in Macagno et al., 2009).

In a preferred embodiment of the aforementioned embodiments, neutralization potency as IC50 is determined in cells infected with any one of HCMV strains TB40/E or VHL/E.

In one preferred embodiment of the aforementioned embodiments, neutralization potency as IC50 is determined in any one of cell lines HFF-1, ARPE-19 or HEC-LTT.

In a preferred embodiment of any of the aforementioned aspects and embodiments, the neutralization potency of the antibody or antigen-binding fragment thereof in fibroblasts, in particular in HFF-1 cells, has an IC50 of at most 150 ng/ml, preferably at most 100 ng/ml, more preferably at most 50 ng/ml.

In another preferred embodiment of any of the aforementioned aspects and embodiments, the neutralization potency of the antibody or antigen-binding fragment thereof in epithelial cells, in particular in ARPE-19 cells, has an IC50 of less than 50 ng/ml, preferably at most 30 ng/ml, more preferably at most 20 ng/ml.

In yet another preferred embodiment of any of the aforementioned aspects and embodiments, the neutralization potency of the antibody or antigen-binding fragment thereof in endothelial cells, in particular HEC-LTT cells, has an IC50 of less than 40 ng/ml, preferably at most 30 ng/ml, more preferably at most 20 ng/ml.

According to a fourth aspect of the present invention, a pharmaceutical composition is provided comprising an antibody or antigen-binding fragment thereof according to any one of the first, second or third aspects of the present invention, and at least one pharmaceutically acceptable excipient.

According to a fifth aspect of the present invention, a kit is provided comprising an antibody or antigen-binding fragment thereof according to any one of the first, second or third aspects of the present invention, and a container.

According to a sixth aspect of the present invention, an antibody or antigen-binding fragment thereof according to any one of the first, second or third aspects, a pharmaceutical composition according to the fourth aspect, or a kit according to the fifth aspect of the present invention is provided for use as a medicament.

According to a seventh aspect of the present invention, an antibody or antigen-binding fragment thereof according to any one of the first, second or third aspects, a pharmaceutical composition according to the fourth aspect, or a kit according to the fifth aspect of the present invention is provided for use in the treatment or prevention of an infection with Human Cytomegalovirus in mammalian subjects, preferably in human subjects.

According to an eighth aspect of the present invention, a nucleic acid is provided encoding an antibody or antigen-binding fragment thereof according to any one of the first, second or third aspects of the present invention.

According to a ninth aspect of the present invention, an expression vector is provided comprising the nucleic acid according to the eighth aspect of the present invention in functional association with an expression control sequence.

According to a tenth aspect of the present invention, a host cell is provided comprising a nucleic acid according to the eighth aspect of the present invention or the expression vector according to the ninth aspect of the present invention.

According to an eleventh aspect of the present invention, a method of production of an antibody or antigen-binding fragment thereof according to any one of the first, second or third aspects of the present invention is provided, comprising cultivating the host cell according to the tenth aspect of the present invention under conditions allowing expression of the antibody or antigen-binding fragment thereof, and recovering the antibody or antigen-binding fragment thereof.

### Brief description of the figures

The present disclosure will be more readily appreciated by reference to the following detailed description when being considered in connection with the accompanying drawings as follows:

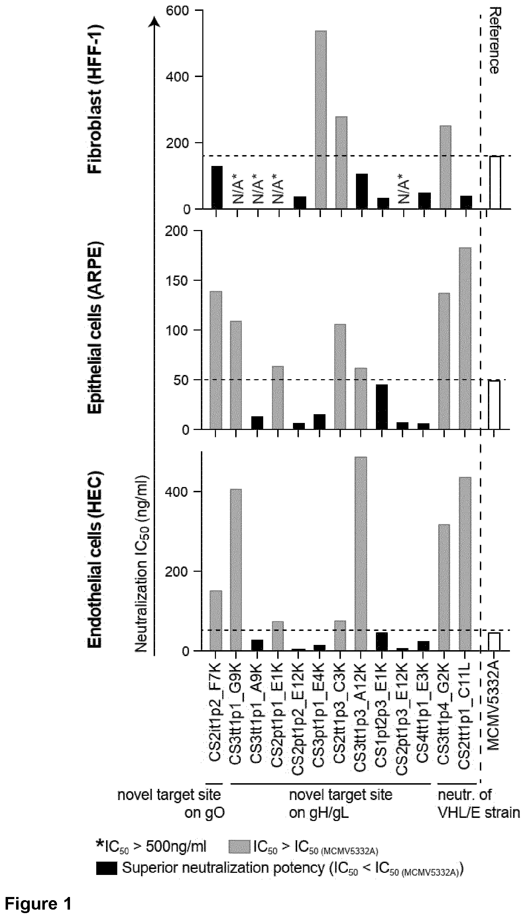

Figure 1 shows neutralization data of selected antibodies of the invention against HCMV_{TB40/E}; neutralization data were determined by preincubation of HCMV_{TB40/E} with different antibody concentrations; antibody/HCMV_{TB40/E} mixture was added to respective target cells (15.000 cells/well) and incubated for 12hrs; afterwards, cells were fixed by acetone and IE-HCMV antigen was stained by fluorescence labeled antibodies; fraction of infected cells was determined by microscopy and IC₅₀ values were calculated; IC₅₀ values are compared to reference antibody MCMV5332A interacting with gH/gL and part of antibody cocktail RG7667.

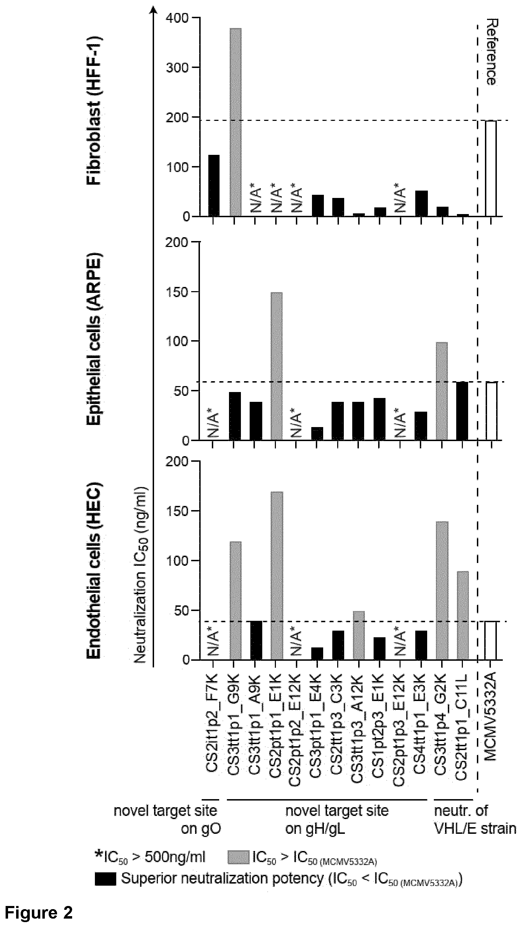

Figure 2 shows neutralization data of selected antibodies of the invention against HCMV_{VHL/E}; neutralization data were determined by preincubation of HCMV_{VHL/E} with different antibody concentrations; antibody/HCMV_{VHL/E} mixture was added to respective target cells (15.000 cells/well) and incubated for 12hrs; afterwards, cells were fixed by acetone and IE-HCMV antigen was stained by fluorescence labeled antibodies; fraction of infected cells was determined by microscopy and IC₅₀ values were calculated; IC₅₀ values are compared to reference antibody MCMV5332A interacting with gH/gL and part of antibody cocktail RG7667.

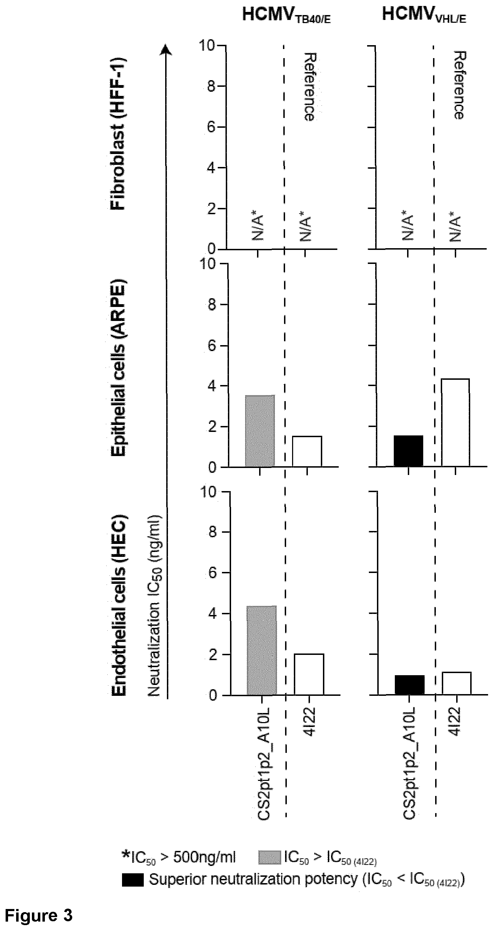

Figure 3 shows neutralization data of the pentamer-specific antibody CS2pt1p2_A10L binding to a novel epitope within HCMV; neutralization data were determined by preincubation of HCMV_{TB40/E} or HCMV_{VHL/E} with different antibody concentrations; antibody/HCMV mixture was added to respective target cells (15.000 cells/well) and incubated for 12hrs; afterwards, cells were fixed by acetone and IE-HCMV antigen was stained by fluorescence labeled antibodies; fraction of infected cells was determined by microscopy and IC₅₀ values were calculated; IC₅₀ values are compared to reference antibody 4I22 interacting with the pentamer-specific subunits UL_{128-131A}.

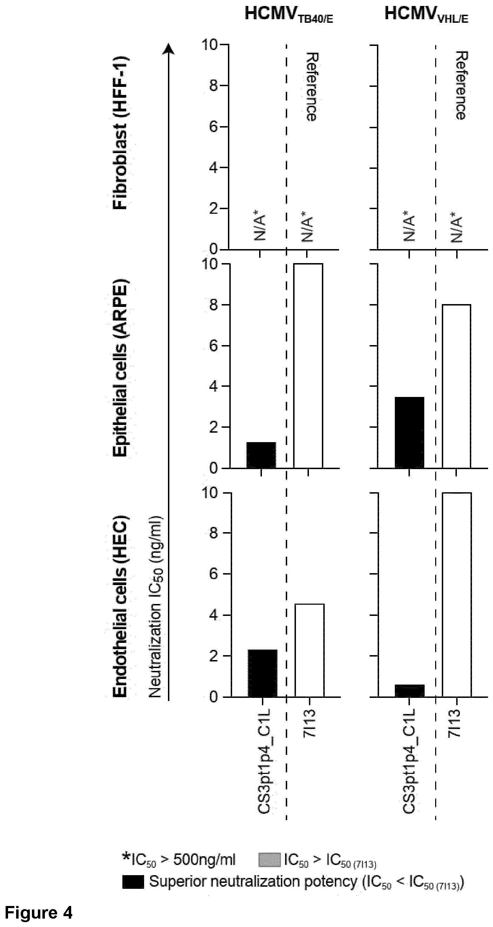

Figure 4 shows neutralization data of the pentamer-specific antibody CS3pt1p4_C1L having superior neutralization activity; neutralization data were determined by preincubation of HCMV_{TB40/E} or HCMV_{VHL/E} with different antibody concentrations; antibody/HCMV mixture was added to respective target cells (15.000 cells/well) and incubated for 12hrs; afterwards, cells were fixed by acetone and IE-HCMV antigen was stained by fluorescence labeled antibodies; fraction of infected cells was determined by microscopy and IC₅₀ values were calculated; IC₅₀ values are compared to reference antibody 7I13 interacting with the pentamer-specific subunits UL_{128-131A} at a similar binding site.

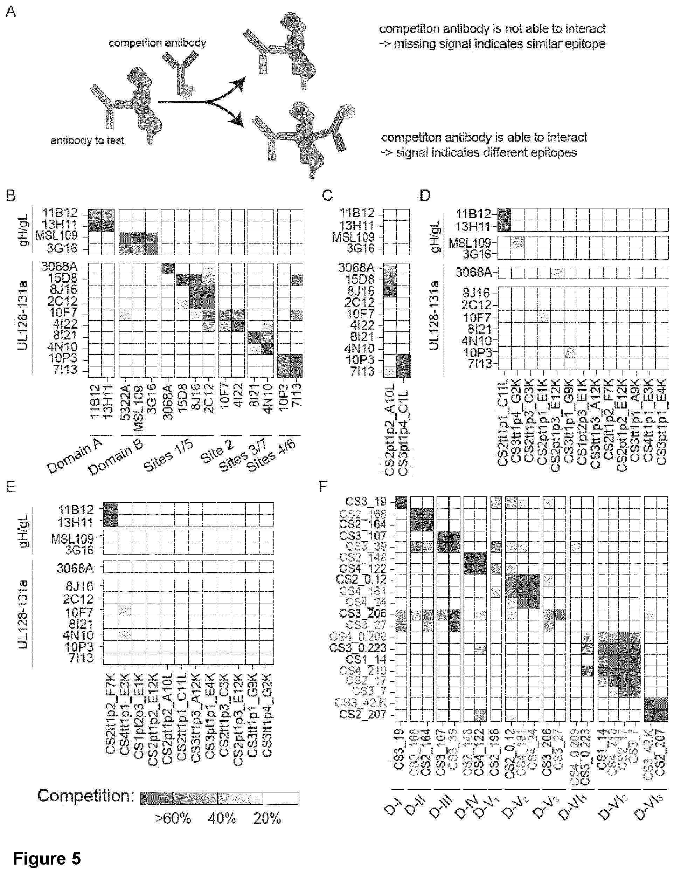

Figure 5 shows data on antibody epitope determination by competition assays; in order to determine antibody binding sites, competition assays were performed; antibodies of interest are incubated with the target glycoprotein and supernatant removed by washing with PBS; thereafter, labeled reference antibodies are added and presence or absence of competition for a binding site determined by signal reduction compared to "antibody of interest only" reference samples; (A) illustration of the experimental setup, (B) competition of reference antibodies against each other; (C) competition of the UL_{128/130/131A}-interacting antibodies of the present invention with reference antibodies; (D) competition of gH/gL-interacting antibodies of the present invention with reference antibodies; (E) competition of gH/gL-interacting antibodies of the present invention with reference antibodies; (F) comparative epitope mapping of antibodies with novel target sites (identified by absence of apparent competition with reference antibodies).

### Detailed description of preferred embodiments

In the following, the invention will be explained in more detail with reference to the accompanying figures. In the Figures, like elements are denoted by identical reference numerals and repeated description thereof may be omitted in order to avoid redundancies.

In order that the present description can be more readily understood, certain terms are first defined. Additional definitions are set forth throughout the detailed description.

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising" otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International d'Unités (SI) accepted form. Numeric ranges are inclusive of the numbers defining the range. Unless otherwise indicated, nucleotide sequences are written left to right in 5' to 3' orientation. Amino acid sequences are written left to right in amino to carboxy orientation. The headings provided herein are not limitations of the various aspects of the disclosure, which can be had by reference to the specification as a whole. Accordingly, the terms defined immediately below are more fully defined by reference to the specification in its entirety.

The term "about" is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, e.g., 10 percent, up or down (higher or lower).

The term "antibody" is used herein in the broadest sense to refer to molecules with an immunoglobulin-like domain (for example IgG, IgM, IgA, IgD or IgE) and includes monoclonal, recombinant, polyclonal, chimeric, human, humanized, multispecific antibodies, including bispecific antibodies, and heteroconjugate antibodies; a single variable domain (e.g., VH, VHH, VL, domain antibody), antigen binding antibody fragments, Fab, F(ab')2, Fv, disulphide linked Fv, single chain Fv, disulphide-linked scFv, diabodies, etc. and modified versions of any of the foregoing.

The term "antibody" as used herein refers to a protein which is capable of specifically binding to an antigen or an antigen-binding portion thereof. The term includes full length antibodies of any class or isotype and any single chain or fragment thereof. An antibody that specifically binds to an antigen, or antigen-binding portion thereof, may bind exclusively to that antigen, or portion thereof, or it may bind to a limited number of homologous antigens, or portions thereof. Full-length antibodies usually comprise at least four polypeptide chains: two heavy (H) chains and two light (L) chains that are interconnected by disulfide bonds.

One immunoglobulin sub-class of particular pharmaceutical interest is the IgG family. In humans, the IgG class may be sub-divided into 4 sub-classes: IgG1, IgG2, IgG3 and IgG4, based on the sequence of their heavy chain constant regions. The light chains can be divided into two types, kappa and lambda, based on differences in their sequence composition. IgG molecules are composed of two heavy chains, interlinked by two or more disulfide bonds, and two light chains, each attached to a heavy chain by a disulfide bond. A heavy chain may comprise a heavy chain variable region (VH) and up to three heavy chain constant (CH) regions: CH1, CH2 and CH3. A light chain may comprise a light chain variable region (VL) and a light chain constant region (CL).

VH and VL regions can be further subdivided into regions of hypervariability, termed complementarity determining regions (CDRs), interspersed with regions that are more conserved, termed framework regions (FR). VH and VL regions are typically composed of three CDRs and four FRs, arranged from amino-terminus to carboxy-terminus in the following order: FR1, CDR1, FR2, CDR2, FR3, CDR3, FR4. The hypervariable regions of the heavy and light chains form a binding domain that is capable of interacting with an antigen, while the constant region of an antibody may mediate binding of the immunoglobulin to host tissues or factors, including but not limited to various cells of the immune system (effector cells), Fc receptors and the first component (C1q) of the classical complement system. Antibodies of the current invention may be isolated.

The term "isolated antibody" refers to an antibody that has been separated and/or recovered from (an)other component(s) in the environment in which it was produced and/or that has been purified from a mixture of components present in the environment in which it was produced. Certain antigen-binding fragments of antibodies may be suitable in the context of the current invention, as it has been shown that the antigen-binding function of an antibody can be performed by fragments of a full-length antibody.

The term "binding fragment" or "antigen-binding fragment" of an antibody refers to one or more fragment(s) of an antibody that retain the ability to specifically bind to an antigen, such as the HCMV as a whole or parts thereof, as described herein.

Examples of antigen-binding fragments include Fab, Fab', F(ab)2, F(ab')2, F(ab)S, Fv (typically the VL and VH domains of a single arm of an antibody), single-chain Fv (scFv; see, e.g., Bird et al., Science 242:42S-426 (1988); Huston et al., PNAS 85: 5879-5883 (1988)), dsFv, Fd (typically the VH and CH1 domain), and dAb (typically a VH domain) fragments; VH, VL, VHH, and V-NAR domains; monovalent molecules comprising a single VH and a single VL chain; minibodies, diabodies, triabodies, tetrabodies, and kappa bodies (see, e.g., III et al., 1997); camel IgG; IgNAR; as well as one or more isolated CDRs or a functional paratope, where the isolated CDRs or antigen-binding residues or polypeptides can be associated or linked together so as to form a functional antibody fragment.

Various types of antibody fragments have been described or reviewed in, e.g., Holliger and Hudson, 2005; International Publ. No. WO 2005/040219, and U.S. Publ. Nos. 2005/0238646 and 2002/0161201. These antibody fragments may be obtained using conventional techniques known to those of skill in the art, and the fragments may be screened for utility in the same manner as intact antibodies.

A "human" antibody (HuMAb) refers to an antibody having variable regions in which both the framework and CDR regions are derived from human germline immunoglobulin sequences. Furthermore, if the antibody contains a constant region, the constant region also is derived from human germline immunoglobulin sequences. The HCMV antibodies described herein can include amino acid residues not encoded by human germline immunoglobulin sequences (e.g., mutations introduced by random or site-specific mutagenesis in vitro or by somatic mutation in vivo).

However, the term "human antibody", as used herein, is not intended to include antibodies in which CDR sequences derived from the germline of another mammalian species, such as a mouse, have been grafted onto human framework sequences. The terms "human" antibodies and "fully human" antibodies are used synonymously.

A "recombinant human antibody" refers to all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom, (b) antibodies isolated from a host cell transformed to express the antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences.

Such recombinant human antibodies comprise variable and constant regions that utilize particular human germline immunoglobulin sequences are encoded by the germline genes, but include subsequent rearrangements and mutations which occur, for example, during antibody maturation. As known in the art (see, e.g., Lonberg, 2005), the variable region contains the antigen binding domain, which is encoded by various genes that rearrange to form an antibody specific for a foreign antigen. In addition to rearrangement, the variable region can be further modified by multiple single amino acid changes (referred to as somatic mutation or hypermutation) to increase the affinity of the antibody to the foreign antigen. The constant region will change in further response to an antigen (i.e., isotype switch).

Therefore, the rearranged and somatically mutated nucleic acid molecules that encode the light chain and heavy chain immunoglobulin polypeptides in response to an antigen cannot have sequence identity with the original nucleic acid molecules, but instead will be substantially identical or similar (i.e., have at least 80% identity).

A "chimeric antibody" refers to an antibody in which the variable regions are derived from one species and the constant regions are derived from another species, such as an antibody in which the variable regions are derived from a mouse antibody and the constant regions are derived from a human antibody.

Alternative antibody formats include alternative scaffolds in which the one or more CDRs of the antigen-binding portion can be arranged onto a suitable non-immunoglobulin protein scaffold or skeleton, such as an affibody, a SpA scaffold, an LDL receptor class A domain, an avimer or an EGF domain.

The term "domain" (interchangeably referred to as "region" herein) refers to a folded protein structure which retains its tertiary structure independent of the rest of the protein. Generally, domains are responsible for discrete functional properties of proteins and in many cases may be added, removed or transferred to other proteins without loss of function of the remainder of the protein and/or of the domain.

The term "variable domain" refers to a folded polypeptide domain comprising sequences characteristic of antibody variable domains. It, therefore, includes complete antibody variable domains such as VH, VHH and VL and modified antibody variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

A single variable (V) domain is capable of binding an antigen or epitope independently of a different variable region or domain. A "domain antibody" or "dAbTM" may be considered the same as a "single variable domain". A single variable domain may be a human single variable domain, but also includes single variable domains from other species such as rodent nurse shark and Camelid VHH dAbsTM. Camelid VHH are immunoglobulin single variable domain polypeptides that are derived from species including camel, llama, alpaca, dromedary, and guanaco, which produce heavy chain antibodies naturally devoid of light chains. Such VHH domains may be humanized according to standard techniques available in the art, and such domains are considered to be "single variable domains".

An antigen-binding fragment may be provided by means of arrangement of one or more CDRs on non-antibody protein scaffolds. "Protein Scaffold" may include an immunoglobulin (Ig) scaffold, for example an IgG scaffold, which may be a four chain or two chain antibody, or which may comprise only the Fc region of an antibody, or which may comprise one or more constant regions from an antibody, which constant regions may be of human or primate origin, or which may be an artificial chimera of human and primate constant regions.

Phrases like "an antibody recognizing an antigen" and "an antibody specific for an antigen" may be used interchangeably herein with the term "an antibody which binds specifically to an antigen."

By the terms "treat," "treating," or "treatment of" (or grammatically equivalent terms) it is meant that the severity of the subject's condition is reduced or at least partially improved or ameliorated and/or that some alleviation, mitigation or decrease in at least one clinical symptom is achieved and/or there is a delay in the progression of the condition.

As used herein, the terms "prevent," "prevents," or "prevention" and "inhibit," "inhibits," or "inhibition" (and grammatical equivalents thereof) are not meant to imply complete abolition of disease and encompasses any type of prophylactic treatment that reduces the incidence of the condition, delays the onset of the condition, and/or reduces the symptoms associated with the condition after onset.

An "effective," "prophylactically effective," or "therapeutically effective" amount as used herein is an amount that is sufficient to provide some improvement or benefit to the subject. Alternatively stated, an "effective," "prophylactically effective," or "therapeutically effective" amount is an amount that will provide some delay, alleviation, mitigation, or decrease in at least one clinical symptom in the subject. Those skilled in the art will appreciate that the effects need not be complete or curative, as long as some benefit is provided to the subject.

A "neutralizing antibody" may refer to any antibody or antigen-binding fragment thereof that binds to a pathogen and interferes with the ability of the pathogen to infect a cell and/or cause disease in a subject.

For polypeptides, the term "substantial homology" indicates that two polypeptides, or designated sequences thereof, when optimally aligned and compared, are identical, with appropriate amino acid insertions or deletions, in at least about 80% of the amino acids, at least about 90% to 95%, or at least about 98% to 99.5% of the amino acids.

The percent identity between two sequences is a function of the number of identical positions shared by the sequences (i.e., % homology = # of identical positions/total # of positions times 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The nucleic acids can be present in whole cells, in a cell lysate, or in a partially purified or substantially pure form. A nucleic acid is "isolated" or "rendered substantially pure" when purified away from other cellular components or other contaminants, e.g., other cellular nucleic acids (e.g., the other parts of the chromosome) or proteins, by standard techniques, including alkaline/SDS treatment, CsCI banding, column chromatography, agarose gel electrophoresis and others well known in the art. See, Ausubel et al., 1987.

Nucleic acids, e.g., cDNA, can be mutated, in accordance with standard techniques to provide gene sequences. For coding sequences, these mutations, can affect amino acid sequence as desired. In particular, DNA sequences substantially homologous to or derived from native V, D, J, constant, switches and other such sequences described herein are contemplated (where "derived" indicates that a sequence is identical or modified from another sequence).

The term "vector," as used herein, is intended to refer to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA or RNA segments can be ligated into the viral genome. Certain vectors are capable of autonomous replication in a host cell into which they are introduced (e.g., bacterial vectors having a bacterial origin of replication and episomal mammalian vectors).

Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" can be used interchangeably as the plasmid is the most commonly used form of vector. However, also included are other forms of expression vectors, such as viral vectors (e.g., replication defective retroviruses, adenoviruses and adeno-associated viruses), which serve equivalent functions.

The term "recombinant host cell" (or simply "host cell"), as used herein, is intended to refer to a cell that comprises a nucleic acid that is not naturally present in the cell, and can be a cell into which a recombinant expression vector has been introduced. It should be understood that such terms are intended to refer not only to the particular subject cell but to the progeny of such a cell. Because certain modifications can occur in succeeding generations due to either mutation or environmental influences, such progeny cannot, in fact, be identical to the parent cell, but are still included within the scope of the term "host cell" as used herein.

As used herein, the term "linked" refers to the association of two or more molecules. The linkage can be covalent or non-covalent. The linkage also can be genetic (i.e., recombinantly fused). Such linkages can be achieved using a wide variety of art recognized techniques, such as chemical conjugation and recombinant protein production.

An "Fc receptor" or "FcR" is a receptor that binds to the Fc region of an immunoglobulin. FcRs that bind to an IgG antibody comprise receptors of the FcγR family, including allelic variants and alternatively spliced forms of these receptors. The FcγR family consists of three activating (FcγRI, FcyRIII, and Fc.RIV in mice; FcyRIA, FcyRIIA, and FcγRIIIA in humans) and one inhibitory (FcγRIIB) receptor. Various properties of human FcyRs are known in the art. The majority of innate effector cell types coexpress one or more activating FcγR and the inhibitory FcγRIIB, whereas natural killer (NK) cells selectively express one activating Fc receptor (FcyRIII in mice and FcγRIIIA in humans) but not the inhibitory FcγRIIB in mice and humans. Human IgG1 binds to most human Fc receptors and is considered equivalent to murine IgG2a with respect to the types of activating Fc receptors that it binds to.

"Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (C1q) of the classical complement system. Thus, an Fc region comprises the constant region of an antibody excluding the first constant region immunoglobulin domain (e.g., CH1 or CL).

The constant region may be modified to stabilize the antibody, e.g., to reduce the risk of a bivalent antibody separating into two monovalent VH-VL fragments. For example, in an IgG4 constant region, residue S228 (residue numbering according to the EU index) may be mutated to a proline (P) residue to stabilize inter heavy chain disulphide bridge formation at the hinge (see, e.g., Angal et al., 1993). Antibodies or fragments thereof can also be defined in terms of their complementarity-determining regions (CDRs).

The term "complementarity-determining region" or "hypervariable region", when used herein, refers to the regions of an antibody in which amino acid residues involved in antigen binding are situated. The region of hypervariability or CDRs can be identified as the regions with the highest variability in amino acid alignments of antibody variable domains.

In general, databases can be used for CDR identification such as the Kabat database, the CDRs e.g., being defined as comprising amino acid residues 24-34 (CDR1), 50-59 (CDR2) and 89-97 (CDR3) of the light-chain variable region, and 31-35 (CDR1), 50-65 (CDR2) and 95-102 (CDR3) in the heavy-chain variable region; (Kabat et al. 1991). Alternatively, CDRs can generally be defined as those residues from a "hypervariable loop" (residues 26-33 (L1), 50-52 (L2) and 91-96 (L3) in the light-chain variable region and 26-32 (H1), 53-55 (H2) and 96-101 (H3) in the heavy-chain variable region (Chothia and Lesk, 1987).

The CDR regions of the antibody sequences described herein are preferably defined according to the numbering scheme of IMGT which is an adaptation of the numbering scheme of Chothia (ImMunoGeneTics information system®; Lefranc et al., 1999.; http://imgt.org).

As used herein, the terms "specific binding," "selective binding," "selectively binds," and "specifically binds," refer to antibody binding to an epitope on a predetermined antigen. Preferably, the antibody binds to the predetermined antigen with an affinity that is at least two-fold greater than its affinity for binding to a non-specific antigen (e.g., BSA, casein) other than the predetermined antigen or a closely-related antigen.

The term "binding affinity" herein refers to a measurement of the strength of a non-covalent interaction between two molecules, e.g. an antibody, or fragment thereof, and an antigen. The term "binding affinity" is used to describe monovalent interactions (intrinsic activity).

The binding affinity between two molecules, e.g. an antibody, or fragment thereof, and an antigen, through a monovalent interaction may be quantified by determination of the equilibrium dissociation constant (KD). In turn, KD can be determined by measurement of the kinetics of complex formation and dissociation, e.g. by the SPR method. The rate constants corresponding to the association and the dissociation of a monovalent complex are referred to as the association rate constant ka (or kon) and dissociation rate constant kd (or koff), respectively. KD is related to ka and kd through the equation KD=kd/ka. Following the above definition, binding affinities associated with different molecular interactions, such as comparison of the binding affinity of different antibodies for a given antigen, may be compared by comparison of the KD values for the individual antibody/antigen complexes.

The term "binding specificity" herein refers to the interaction of a molecule such as an antibody, or fragment thereof, with a single exclusive antigen, or with a limited number of highly homologous antigens (or epitopes). In contrast, antibodies that are capable of specifically binding HCMV are not capable of binding dissimilar molecules.

The specificity of an interaction and the value of an equilibrium binding constant can be determined directly by well-known methods. Standard assays to evaluate the ability of ligands (such as antibodies) to bind their targets are known in the art and include, for example, ELISAs, Western blots, RIAs, and flow cytometry analysis. The binding kinetics and binding affinity of the antibody also can be assessed by standard assays known in the art, such as SPR.

A "polypeptide" refers to a chain comprising at least two consecutively linked amino acid residues, with no upper limit on the length of the chain. One or more amino acid residues in the protein can contain a modification such as, but not limited to, glycosylation, phosphorylation or disulfide bond formation. A "protein" can comprise one or more polypeptides.

The term "nucleic acid" or "nucleic acid molecule," as used herein, is intended to include DNA molecules and RNA molecules. A nucleic acid molecule can be single-stranded or double-stranded, and can be cDNA.

The term "subject" includes human and other mammalian subjects that receive either prophylactic or therapeutic treatment. As used herein, the term "subject" includes any human or non-human animal. The term "non-human animal" includes all vertebrates, e.g., mammals and non-mammals, such as non-human primates, sheep, dog, cow, chickens, amphibians, reptiles, etc.

As used herein, the terms "ug" and "uM" are used interchangeably with "µg" and "µM," respectively.

As used herein, "administering" refers to the physical introduction of a composition comprising a therapeutic agent to a subject, using any of the various methods and delivery systems known to those skilled in the art. Different routes of administration for the antibodies described herein include intravenous, intraperitoneal, intramuscular, subcutaneous, spinal or other parenteral routes of administration, for example by injection or infusion.

The phrase "parenteral administration" as used herein means modes of administration other than enteral and topical administration, usually by injection, and includes, without limitation, intravenous, intraperitoneal, intramuscular, intraarterial, intrathecal, intralymphatic, intralesional, intracapsular, intraorbital, intracardiac, intradermal, transtracheal, subcutaneous, subcuticular, intraarticular, subcapsular, subarachnoid, intraspinal, epidural and intrasternal injection and infusion, as well as in vivo electroporation.

Alternatively, an antibody described herein can be administered via a non-parenteral route, such as a topical, epidermal or mucosal route of administration, for example, intranasally, orally, vaginally, rectally, sublingually or topically. Administering can also be performed, for example, once, a plurality of times, and/or over one or more extended periods.

As used herein "vaccination composition" means a pharmaceutical composition comprising at least one antibody or antigen-binding portion thereof of the present invention which is capable of providing active and/or passive immunity. "Active immunity" as used herein means inducing or enhancing a subject's immune response to an antigen. "Passive immunity" as used and preferred herein means supplementing a subject's immune response to an antigen or pathogen by providing antibodies and/or antigen-binding portions thereof which neutralize an antigen.

The present inventors have dedicated themselves to solving the problem of the present invention and were successful to find novel human monoclonal antibodies against *Human Cytomegalovirus* (HCMV; also commonly referred to as *Human Betaherpesvirus 5*) having superior neutralization potency against the virus, and most importantly able to neutralize infection of epithelial cells and endothelial cells as well as to suppress fibroblast infection unline any other antibody of the prior art.

Antibodies of the prior art suffered from several problems including an unsatisfactory neutralization potency and failure to neutralize infections in different cell types. Such weaknesses could potentially be overcome when combining antibodies binding to different target proteins or epitopes which may cause complementary neutralization abilities.

In the context of identifying the novel antibodies according to the present invention, it could further be determined by competition experiments with antibodies of the prior art that certain of these inventive antibodies bind to novel, previously unrecognized epitopes of the infection machinery of HCMV (cf. Figure 5).

It is well known in the art that any antibodies binding to epitopes that are different to the ones recognized by antibodies of the prior art can be used to improve virus neutralization when combined with antibodies of the prior art. Accordingly, antibodies binding to such epitopes enable the skilled person to identify these epitopes, which in turn enables the skilled person to generate additional antibodies specifically binding to these epitopes by using technology commonly known in the art, such as antibody phage display or hybridoma technology.

The present inventors addressed the need for human monoclonal antibodies with improved neutralization strength, novel binding targets and different effects observed on HCMV infection in different cell types.

To this end, the inventors screened 9,000 HCMV-seropositive blood donors, identified 58 individuals with highly potent HCMV neutralizing activity and finally arrived at four different donors which exhibited a desired response profile to likely be able to provide promising monoclonal HCMV antibodies of interest. From these donors, 146 monoclonal antibodies were isolated and studied further.

Of these, 136 monoclonal antibodies (mAbs) interacted with the Dimer, while nine mAbs were found to depend on the UL128/130/131A-subunits of the Pentamer complex, and one mAb on the gO-subunit of the Trimer. The Trimer complex (made up of gH/gL/gO) and the Pentamer complex (made up of gH/gL/UL128/130/131A) of HCMV are essential for viral entry into host cells. The Dimer is an artificially created, soluble construct comprised of the two subunits common to Trimer and Pentamer (gH/gL).

These antibodies were then further screened for general neutralization capability, ability to prevent epithelial, endothelial and fibroblast cells. Also, antibodies were examined in competition assays with each other and with antibodies of the prior art to find out if certain antibodies may bind to previously unrecognized target epitopes of HCMV (cf. Figure 5). These new broadly neutralizing human monoclonal antibodies may hold great therapeutic or prophylactic potential.

The antibodies of the present invention comprise novel monoclonal antibodies against HCMV that interact with novel target sites essential for HCMV infection, and demonstrate superior neutralizing activity compared to current antibodies in clinical trials. These inventive antibodies have been isolated from individuals exhibiting exceptional neutralization activity against fibroblasts, which individuals are particularly vulnerable to CMV infection because they often suffer from inadequate protection by the host induced immune response.

In particular, CS2it1p2_F7K interacts with the gO subunit of the trimer, which plays an essential role during fibroblast infection and is assumed to be involved in HCMV infection of all other cell types. As it binds to a subunit for which only a single antibody has been mentioned in the literature before (however without any disclosure of its CDR or any other amino acid sequence, and thus not made available to the public), and none are available in clinical studies, this antibody is an ideal candidate for future monotherapies as well as antibody cocktail approaches.

The second key aspect of the present invention involves antibodies that target the gH/gL complex of CMV. Although gH/gL does not show direct receptor interaction, the inventors have identified antibodies that bind to new, previously undescribed epitopes on this complex, and lead to impaired receptor interaction (CS3pt1p1_E4K, CS2pt1p2_E12K, CS2tt1p4_A1K, **CS3tt1**p1_A9K, CS3tt1p2_B1K blocking PDGFRa and TGFBR3; CS4tt1p1_E3K, CS2tt1p2_G2K blocking TGFBR3 only, and CS2pt1p1_E1K blocking NRP2).

This achievement has not been attained by any of the previously described antibodies that bind to two other binding sites on gH/gL. Furthermore, the inventors have identified antibodies that interact with additional previously undescribed epitopes on gH/gL. Although these antibodies do not show receptor competition, they exhibit high to exceptional neutralization potency (CS2tt1 p3_C3K, CS2pt1 p1_E1K, CS4tt1 p3_A7K, CS3tt1 p1_G9K, CS3tt1 p1_A4K, CS3tt1 p3_A12K, CS4tt1 p2_C3L, S1 pt2p3_E1K, CS2tt1 p1_B12K, CS4pt1 p2_A6L, CS3tt1 p1_B5K, CS2pt1 p3_E12K, CS3pt1 p1_C5K).

In addition to the inventive antibodies interacting with new binding sites as described herein, it should be emphasized that many of them not only exhibit excellent neutralization but also outperform comparable known antibodies involved in clinical studies in protecting fibroblasts, epithelial cells, and/or endothelial cells (CS4tt1 p1_E3K, CS2pt1 p2_E12K, CS2tt1 p1_B12K, CS1 pt2p3_E1K, CS3tt1 p3_A12K, CS2tt1 p3_C3K, CS3pt1 p1_E4K, CS2pt1 p3_E12K, CS2tt1 p4_A1K, CS3pt1 p1_C5K).

Of particular note is CS4tt1 p1_E3K, which as a single antibody exhibits comparable to slightly superior neutralization efficacy compared to the clinically tested antibody cocktail RG7667, owing to its broad reactivity.

In addition to their direct therapeutic potential for the use in various combination therapies, these newly recognized target sites combined with their associated antibodies disclosed herein will provide an important basis for advantageous therapeutic approaches and the identification of new strategies for HCMV infection and prevention.

For example, although it is widely recognized that gH/gL is important for the activation of the fusion protein gB, this has not been definitively proven thus far. However, with the antibodies of the present invention which target novel surface structures on gH/gL, such investigation can be carried out in more detail.

Furthermore, the present invention further encompasses three antibodies that recognize the previously known antibody target sites on gH/gL but exhibit 9 to 20 times greater neutralization capacity against certain HCMV strains in fibroblasts than the previously described antibodies with comparable binding sites.

Moreover, this invention comprises two additional antibodies against the pentameric complex. In this regard, we have identified an antibody (CS2pt1 p2_A10L) that exhibits comparable neutralization activity to the most potent clinically tested antibodies, but recognizes a new epitope, making it ideal for combination therapies to prevent escape mutations and obtain additive effects with other antibodies.

In addition, due to its novel epitope, this antibody is suitable for inhibiting an important step of HCMV infection, namely, the dimerization of the pentamer during THBC-receptor interaction. Another candidate targeting the pentamer (CS3pt1p4_C1L) demonstrates superior activity in protecting endothelial and epithelial cells compared to the antibodies of the prior art MCMV3068A and 4I22 which are currently used in clinical studies.

Finally, it is worth noting that the antibodies CS2it1p2_F7K, CS4tt1p1_E3K, CS2pt1p2_A10L, and CS3pt1p4_C1L have been tested for their suitability as combination preparations. In this regard, a fully additive effect leading up to even synergistic effects was observed in the protection of epithelial cells, as well as, where applicable, for fibroblasts. These findings provide ideal requirements for an application in therapy or prevention of CMV infection and underscore the suitability of the listed antibodies for future assessment in clinical studies.

In conclusion, this invention provides novel monoclonal antibodies against HCMV that exhibit superior neutralizing activity compared to current antibodies in clinical trials. These antibodies target novel epitopes essential for HCMV infection, offering potential for combination therapies and a deeper understanding of HCMV infection mechanisms.

Furthermore, they demonstrate exceptional neutralization potency and surpass known antibodies in protecting fibroblasts, epithelial cells, and/or endothelial cells. The invention also includes antibodies that recognize previously known target sites but possess significantly enhanced neutralization capabilities against specific HCMV strains in fibroblasts. Additionally, antibodies targeting the pentameric complex show comparable or superior neutralization activity while recognizing new epitopes, making them valuable candidates for combination therapies and preventing escape mutations.

Accordingly, the present invention provides antibodies or antigen-binding fragments directed against *Human Cytomegalovirus (HCMV; Human Betaherpesvirus 5)* is provided, wherein the antibody or antigen-binding fragment thereof comprises the combination of the heavy chain CDR1 to CDR3 and the light chain CDR1 to CDR3 amino acid sequence of one antibody selected from the group comprising **CS2it1p2_F7K** (having a CDR-H1 amino acid sequence of SEQ ID No. 1, a CDR-H2 amino acid sequence of SEQ ID No. 2, a CDR-H3 amino acid sequence of SEQ ID No. 3, a CDR-L1 amino acid sequence of SEQ ID No. 4, a CDR-L2 amino acid sequence of SEQ ID No. 5, a CDR-L3 amino acid sequence of SEQ ID No. 6), **CS4tt1p1_E3K** (having a CDR-H1 amino acid sequence of SEQ ID No. 7, a CDR-H2 amino acid sequence of SEQ ID No. 8, a CDR-H3 amino acid sequence of SEQ ID No. 9, a CDR-L1 amino acid sequence of SEQ ID No. 10, a CDR-L2 amino acid sequence of SEQ ID No. 11, a CDR-L3 amino acid sequence of SEQ ID No. 12), **CS1pt2p3_E1K** (having a CDR-H1 amino acid sequence of SEQ ID No. 13, a CDR-H2 amino acid sequence of SEQ ID No. 14, a CDR-H3 amino acid sequence of SEQ ID No. 15, a CDR-L1 amino acid sequence of SEQ ID No. 16, a CDR-L2 amino acid sequence of SEQ ID No. 17, a CDR-L3 amino acid sequence of SEQ ID No. 18), **CS2pt1p2_E12K** (having a CDR-H1 amino acid sequence of SEQ ID No. 19, a CDR-H2 amino acid sequence of SEQ ID No. 20, a CDR-H3 amino acid sequence of SEQ ID No. 21, a CDR-L1 amino acid sequence of SEQ ID No. 22, a CDR-L2 amino acid sequence of SEQ ID No. 23, a CDR-L3 amino acid sequence of SEQ ID No. 24), **CS2pt1p2_A10L** (having a CDR-H1 amino acid sequence of SEQ ID No. 25, a CDR-H2 amino acid sequence of SEQ ID No. 26, a CDR-H3 amino acid sequence of SEQ ID No. 27, a CDR-L1 amino acid sequence of SEQ ID No. 28, a CDR-L2 amino acid sequence of SEQ ID No. 29, a CDR-L3 amino acid sequence of SEQ ID No. 30), **CS2tt1p1_C11L** (having a CDR-H1 amino acid sequence of SEQ ID No. 31, a CDR-H2 amino acid sequence of SEQ ID No. 32, a CDR-H3 amino acid sequence of SEQ ID No. 33, a CDR-L1 amino acid sequence of SEQ ID No. 34, a CDR-L2 amino acid sequence of SEQ ID No. 35, a CDR-L3 amino acid sequence of SEQ ID No. 36), **CS3tt1p3_A12K** (having a CDR-H1 amino acid sequence of SEQ ID No. 37, a CDR-H2 amino acid sequence of SEQ ID No. 38, a CDR-H3 amino acid sequence of SEQ ID No. 39, a CDR-L1 amino acid sequence of SEQ ID No. 40, a CDR-L2 amino acid sequence of SEQ ID No. 41, a CDR-L3 amino acid sequence of SEQ ID No. 42), **CS3pt1p1_E4K** (having a CDR-H1 amino acid sequence of SEQ ID No. 43, a CDR-H2 amino acid sequence of SEQ ID No. 44, a CDR-H3 amino acid sequence of SEQ ID No. 45, a CDR-L1 amino acid sequence of SEQ ID No. 46, a CDR-L2 amino acid sequence of SEQ ID No. 47, a CDR-L3 amino acid sequence of SEQ ID No. 48), **CS2tt1p3_C3K** (having a CDR-H1 amino acid sequence of SEQ ID No. 49, a CDR-H2 amino acid sequence of SEQ ID No. 50, a CDR-H3 amino acid sequence of SEQ ID No. 51, a CDR-L1 amino acid sequence of SEQ ID No. 52, a CDR-L2 amino acid sequence of SEQ ID No. 53, a CDR-L3 amino acid sequence of SEQ ID No. 54), **CS2pt1p3_E12K** (having a CDR-H1 amino acid sequence of SEQ ID No. 55, a CDR-H2 amino acid sequence of SEQ ID No. 56, a CDR-H3 amino acid sequence of SEQ ID No. 57, a CDR-L1 amino acid sequence of SEQ ID No. 58, a CDR-L2 amino acid sequence of SEQ ID No. 59, a CDR-L3 amino acid sequence of SEQ ID No. 60), **CS3tt1p1_G9K** (having a CDR-H1 amino acid sequence of SEQ ID No. 61, a CDR-H2 amino acid sequence of SEQ ID No. 62, a CDR-H3 amino acid sequence of SEQ ID No. 63, a CDR-L1 amino acid sequence of SEQ ID No. 64, a CDR-L2 amino acid sequence of SEQ ID No. 65, a CDR-L3 amino acid sequence of SEQ ID No. 66), **CS3tt1p4_G2K** (having a CDR-H1 amino acid sequence of SEQ ID No. 67, a CDR-H2 amino acid sequence of SEQ ID No. 68, a CDR-H3 amino acid sequence of SEQ ID No. 69, a CDR-L1 amino acid sequence of SEQ ID No. 70, a CDR-L2 amino acid sequence of SEQ ID No. 71, a CDR-L3 amino acid sequence of SEQ ID No. 72), **CS3tt1p1_A9K** (having a CDR-H1 amino acid sequence of SEQ ID No. 73, a CDR-H2 amino acid sequence of SEQ ID No. 74, a CDR-H3 amino acid sequence of SEQ ID No. 75, a CDR-L1 amino acid sequence of SEQ ID No. 76, a CDR-L2 amino acid sequence of SEQ ID No. 77, a CDR-L3 amino acid sequence of SEQ ID No. 78), and **CS2pt1p1_E1K** (having a CDR-H1 amino acid sequence of SEQ ID No. 79, a CDR-H2 amino acid sequence of SEQ ID No. 80, a CDR-H3 amino acid sequence of SEQ ID No. 81, a CDR-L1 amino acid sequence of SEQ ID No. 82, a CDR-L2 amino acid sequence of SEQ ID No. 83, a CDR-L3 amino acid sequence of SEQ ID No. 84).

Also, one preferred embodiment of the present invention provides antibodies or antigen-binding fragments directed against Human Cytomegalovirus (HCMV; Human Betaherpesvirus 5), wherein the antibody or antigen-binding fragment thereof comprises the combination of the heavy chain CDR1 to CDR3 and the light chain CDR1 to CDR3 amino acid sequence of the antibody **CS3pt1p4_C1L** (having a CDR-H1 amino acid sequence of SEQ ID No. 113, a CDR-H2 amino acid sequence of SEQ ID No. 114, a CDR-H3 amino acid sequence of SEQ ID No. 115, a CDR-L1 amino acid sequence of SEQ ID No. 116, a CDR-L2 amino acid sequence of SEQ ID No. 117, a CDR-L3 amino acid sequence of SEQ ID No. 118).

Within the context of the present invention, the antibodies, which have been generated and described herein, may be used and claimed as the complete monoclonal human antibody or as any functional or antigen-binding fragment thereof. Preferably, the antibody or any kind of functional or antigen-binding fragment thereof should at least comprise the CDR 1 to 3 of the heavy chain and CDR 1 to 3 of the light chain of the antibody.

The CDR regions of the antibody sequences described herein are preferably defined according to the numbering scheme of IMGT which is an adaptation of the numbering scheme of Chothia (ImMunoGeneTics information system^{®}; Lefranc et al., 1999; http://imgt.org).

Based on the common general knowledge and the information given herein on the heavy chain variable region amino acid sequences and the light chain variable region amino acid sequences of the antibodies of the invention, the CDRs can be easily and unambiguously determined by a skilled person.

According to the present invention, the CDR sequences of the light and heavy chain variable region sequences of the antibodies and antigen-binding fragments thereof described herein are as follows:

| **Source** | **CDR1** | **SEQ ID CDR1** | **CDR2** | **SEQ ID CDR2** | **CDR3** | **SEQ ID CDR3** |
|---|---|---|---|---|---|---|
| Heavy chain CS2it1p2_F7K | GYTLTDLS | 1 | FDPEHGEI | 2 | ATDSSIAMTGT YFSDLFALDV | 3 |
| Light chain CS2it1p2_F7K | QTINTN | 4 | GTF | 5 | QQSHSPPHT | 6 |
| Heavy chain CS4tt1p1_E3K | GYTFDQYS | 7 | ISSNGGSR | 8 | ARAKKIFGGIIP PSGMDV | 9 |
| Light chain CS4tt1p1_E3K | QNIYNW | 10 | DAS | 11 | QHYYNYPPWT | 12 |
| Heavy chain CS1pt2p3_E1K | GYSFNTYP | 13 | INTYKGDT | 14 | AKEDPWGWLE Y | 15 |
| Light chain CS1pt2p3_E1K | QGISSY | 16 | GAS | 17 | QQYSTYAYT | 18 |
| Heavy chain CS2pt1 p2_E12K | GESLTDKC | 19 | IDHMDTT | 20 | ARGGPSLKTV YGVVFLHNFF DT | 21 |
| Light chain CS2pt1p2_E12K | QSLLYRSENK NY | 22 | WAS | 23 | QQYYRAPWT | 24 |
| Heavy chain CS2pt1p2_A10L | GFSFSDYY | 25 | ISPSGSPT | 26 | ARDNTVFGVV TTPMDH | 27 |
| Light chain CS2pt1p2_A10L | SSNIGSNT | 28 | SNN | 29 | AAWDDSLNGR V | 30 |
| Heavy chain CS2tt1p1_C11L | 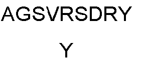 | 31 | LYFGGTN | 32 | AAALTTVTFIS | 33 |
| Light chain CS2tt1p1_C11L | SGINVGTYR | 34 | YKSDSDK | 35 | MIWHNSVVV | 36 |
| Heavy chain CS3tt1p3_A12K | GFTFTDYY | 37 | ISSSGSYT | 38 | ARSPMKRLDF | 39 |
| Light chain CS3tt1p3_A12K | QSVGST | 40 | GTS | 41 | QHYESWPPW T | 42 |
| Heavy chain CS3pt1p1_E4K | GYTFSNYG | 43 | ISGYNGNT | 44 | AREGSRPYYY RGMDV | 45 |
| Light chain CS3pt1p1_E4K | QSISSY | 46 | DTV | 47 | QQRGMSLPW T | 48 |
| Heavy chain CS2tt1p3_C3K | GGSISNYY | 49 | IYYSGST | 50 | ARQRTPNYYG SGSFSNWFDP | 51 |
| Light chain CS2tt1p3_C3K | QSVLFSSNN KNY | 52 | WAS | 53 | QQYYGLPST | 54 |
| Heavy chain CS2pt1p3_E12K | GDSVSNNDA A | 55 | THYRSRW YN | 56 | ARGTPSPKDY SDGGGYYYVE YFQD | 57 |
| Light chain CS2pt1p3_E12K | QSVSTD | 58 | DAS | 59 | QQRTNWPLAY T | 60 |
| Heavy chain CS3tt1p1_G9K | GYTFSNYW | 61 | IYPGDSDT | 62 | GLGLAAGGRF YYYYHGMDV | 63 |
| Light chain CS3tt1p1_G9K | 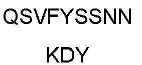 | 64 | WAS | 65 | QQYYTTPQ | 66 |
| Heavy chain CS3tt1p4_G2K | GFTFSDYY | 67 | ISSASSYI | 68 | ARSPMKVLDH | 69 |
| Light chain CS3tt1p4_G2K | QSVDNR | 70 | GAS | 71 | | 72 |
| Heavy chain CS3tt1p1_A9K | GFSFSAYA | 73 | IWFDGSK K | 74 | SREEYRSFNY | 75 |
| Light chain CS3tt1p1_A9K | RSIGTN | 76 | DAS | 77 | QQSYSTPRT | 78 |
| Heavy chain CS2pt1p1_E1K | GRSMNDFY | 79 | IYYTGST | 80 | ARQRTPNYFA SGTYSNWFDP | 81 |
| Light chain CS2pt1p1_E1K | QSLLYSANNK NY | 82 | WAS | 83 | QQYFATPPT | 84 |
| Heavy chain CS3pt1p4_C1L | GGTFTDYA | 113 | IIPVLGTP | 114 | ARGVTHPYYY YAMDV | 115 |
| Light chain CS3pt1p4_C1L | SSDVGAYDY | 116 | DVN | 117 | GSYTSSSAFFY V | 118 |

According to one preferred embodiment of the present invention, the antibody or antigen-binding fragment thereof comprises the combination of the variable region heavy chain amino acid sequence and of the variable region light chain amino acid sequence of one antibody selected from the group comprising CS2it1p2_F7K (having the variable region heavy chain amino acid sequence of SEQ ID No. 85 and the variable region light chain amino acid sequence of SEQ ID No. 86), CS4tt1p1_E3K (having the variable region heavy chain amino acid sequence of SEQ ID No. 87 and the variable region light chain amino acid sequence of SEQ ID No. 88), CS1pt2p3_E1K (having the variable region heavy chain amino acid sequence of SEQ ID No. 89 and the variable region light chain amino acid sequence of SEQ ID No. 90), CS2pt1p2_E12K (having the variable region heavy chain amino acid sequence of SEQ ID No. 91 and the variable region light chain amino acid sequence of SEQ ID No. 92), CS2pt1p2_A10L (having the variable region heavy chain amino acid sequence of SEQ ID No. 93 and the variable region light chain amino acid sequence of SEQ ID No. 94), CS2tt1p1_C11L (having the variable region heavy chain amino acid sequence of SEQ ID No. 95 and the variable region light chain amino acid sequence of SEQ ID No. 96), CS3tt1p3_A12K (having the variable region heavy chain amino acid sequence of SEQ ID No. 97 and the variable region light chain amino acid sequence of SEQ ID No. 98), CS3pt1p1_E4K (having the variable region heavy chain amino acid sequence of SEQ ID No. 99 and the variable region light chain amino acid sequence of SEQ ID No. 100), CS2tt1p3_C3K (having the variable region heavy chain amino acid sequence of SEQ ID No. 101 and the variable region light chain amino acid sequence of SEQ ID No. 102), CS2pt1p3_E12K (having the variable region heavy chain amino acid sequence of SEQ ID No. 103 and the variable region light chain amino acid sequence of SEQ ID No. 104), CS3tt1p1_G9K (having the variable region heavy chain amino acid sequence of SEQ ID No. 105 and the variable region light chain amino acid sequence of SEQ ID No. 106), CS3tt1p4_G2K (having the variable region heavy chain amino acid sequence of SEQ ID No. 107 and the variable region light chain amino acid sequence of SEQ ID No. 108), CS3tt1p1_A9K (having the variable region heavy chain amino acid sequence of SEQ ID No. 109 and the variable region light chain amino acid sequence of SEQ ID No. 110), and CS2pt1p1_E1K (having the variable region heavy chain amino acid sequence of SEQ ID No. 111 and the variable region light chain amino acid sequence of SEQ ID No. 112).

Also, one preferred embodiment of the present invention provides antibodies or antigen-binding fragments directed against Human Cytomegalovirus (HCMV; Human Betaherpesvirus 5), wherein the antibody or antigen-binding fragment thereof comprises the combination of the variable region heavy chain amino acid sequence and of the variable region light chain amino acid sequence of the antibody CS3pt1p4_C1L (having the variable region heavy chain amino acid sequence of SEQ ID No. 119 and the variable region light chain amino acid sequence of SEQ ID No. 120).

According to the present invention, the light and heavy chain variable region sequences of the preferred antibodies and antigen-binding fragments thereof described herein with the internal designations CS2it1 p2_F7K, CS4tt1 p1_E3K, CS1 pt2p3_E1K, CS2pt1 p2_E12K, CS2pt1 p2_A10L, CS2tt1 p1_C11L, CS3tt1 p3_A12K, CS3pt1 p1_E4K, CS2tt1 p3_C3K, CS2pt1 p3_E12K, CS3tt1 p1_G9K, CS3tt1 p4_G2K, CS3tt1 p1_A9K, CS2pt1 p1_E1K, and CS3pt1 p4_C1L are as follows:

| **SEQ ID NO** | **Source** | **Sequence** |
|---|---|---|
| 85 | Heavy chain variable domain CS2it1p2_F7K | QVQLVQSGAEVKKPGASVRVSCKVSGYTLTDLSIHWVRQAPGK GLEWMGGFDPEHGEIMYAQKFQGRVTVTEDTSTHTTYMEVNSL RSEDTAVYYCATDSSIAMTGTYFSDLFALDVWGQGTTVIVSS |
| 86 | Light chain variable domain CS2it1p2_F7K | DIQMTQSPSSLSASVGDRVTITCRASQTINTNLNWYQQKPGKAP RLLIFGTFSLKSGVPSRFSGGGSGTDFTLTISSLQPEDFAAYFCQ QSHSPPHTFGQGTKLELK |
| 87 | Heavy chain variable domain CS4tt1p1_E3K | EVRLVESGGGFVQTGGSLRLSCAASGYTFDQYSMHWVRQVPG KGLQFVSTISSNGGSRYYADSVKGRFVVSRDEEKEVLYLQMGR LRTDDTGIYFCARAKKIFGGIIPPSGMDVWGRGTTVTVSS |
| 88 | Light chain variable domain CS4tt1p1_E3K | DIQLTQSPSSLSASVGDRVTITCRASQNIYNWLAWYQQKPGQAP KLLIFDASTLEGGVPSRFGGSGSGTDFTLTISNLRPEDFATYFCQ HYYNYPPWTFGQGTKV |
| 89 | Heavy chain variable domain CS1pt2p3_E1K | QVQLVQSGVEVKKPGAAVKVSCKASGYSFNTYPIAWVRQAPGE GLEWIGRINTYKGDTKYLQRLQGRVAMTIDISTATAYMELRSLRS DDTAVYFCAKEDPWGWLEYWGQGTPVTVSS |
| 90 | Light chain variable domain CS1pt2p3_E1K | DIQMTQSPSTLSASVGDRVTITCRASQGISSYLAWYQQKPGKAP NLLIFGASTLQSGVPSRFSGSGSGTDFTLTISSLQPDDFATYYCQ QYSTYAYTFGQGTRLEIK |
| 91 | Heavy chain variable domain CS2pt1p2 E12K | QVQLQQWGAGLLRPSETLSLTCAVYGESLTDKCWTWIRQAPGK GLEWIGDIDHMDTTKYNPSLKSRVTISVDGSKNQFSLRLDSVTAA DTGVYYCARGGPSLKTVYGVVFLHNFFDTWGQGTRVVVSS |
| 92 | Light chain variable domain CS2pt1p2_E12K | DVVMTQSPDSLALSLGERATINCKSSQSLLYRSENKNYLAWYQQ KPGQSPRLLISWASDRHSGVPERFSGSGSDTDFILTINSLQAEDV AVYYCQQYYRAPWTFGQGTKVEVK |
| 93 | Heavy chain variable domain CS2pt1p2 A10L | QVHLEESGGGLVKPGGSLRLSCVASGFSFSDYYMSWIRQAPGK GLEWLSYISPSGSPTSNADSMKGRFTISRDNARSSLYLQVHSLR VEDTAVYYCARDNTVFGVVTTPMDHWGQGTLVTVSS |
| 94 | Light chain variable domain CS2pt1p2_A10L | QSALTQPPSASGTPGQRVTISCSGSSSNIGSNTVHWYQQLPGT APKLLIYSNNQRPSGVPDRFSGSRSGTSASLAISGLRSEDEADY YCAAWDDSLNGRVFGTGTKVTV |
| 95 | Heavy chain variable domain CS2tt1p1_C11L | QVQLQESGPGLMRPSETLSLTCTVSAGSVRSDRYYWTWIRQHP GKGLEWIGYLYFGGTNYYNPSLKSRVTISVDASKNQFSLRLTSVT AADTARYYCAAALTTVTFISWGQGAQVTVA |
| 96 | Light chain variable domain CS2tt1p1_C11L | QAVLTQPSSLSASPGASASLTCTLHSGINVGTYRIYWYQQKPGS PPQYLLRYKSDSDKQRGSGVPSRFSGSKDASANAGRLVISGLQ SEDEADYFCMIWHNSVVVFGGGTKLTVL |
| 97 | Heavy chain variable domain CS3tt1p3_A12K | QVQLVQSGGGLVKPGGSLRLSCAASGFTFTDYYMSWIRQSPGK GLEWISYISSSGSYTFYPDSVKGRFTVSRDNTKNSLFLQMDRLR VEDTAVYYCARSPMKRLDFWGQGVLVTVSS |
| 98 | Light chain variable domain CS3tt1p3_A12K | EIVMTQSPATLSLSPGERATLSCRASQSVGSTLAWYQQKPGQA PRLLIFGTSTRATGVPTRFSGSGSGTEFTLIISSLRSEDSGLYFCQ HYESWPPWTFGQGTKVEV |
| 99 | Heavy chain variable domain CS3pt1p1_E4K | QVHLVQSGAEVKKPGASVKVSCKASGYTFSNYGISWVRQAPGQ GLEWMGWISGYNGNTNYAQKFKDRVIMSTDPFTSTVYLEVRSL RSDDTAVYYCAREGSRPYYYRGMDVWGQGTTVTVSS |
| 100 | Light chain variable domain CS3pt1p1_E4K | EIVLTQFPATLSLSPGERATLFCRASQSISSYVAWYQHKPGQAP RLLIYDTVSRATGVPARFSGAGSGTDFTLSISSLESGDFGVYYCQ QRGMSLPWTFGQGTKVEIK |
| 101 | Heavy chain variable domain CS2tt1p3_C3K | QVQLQESGPGLVKPSETLSLTCTVSGGSISNYYWNWIRQPPGK RLEWIGYIYYSGSTNYNPSLKSRVSIAVDTSKKQFSLKLSSVTAA DTAVYYCARQRTPNYYGSGSFSNWFDPWGQGTLVTVSS |
| 102 | Light chain variable domain CS2tt1p3_C3K | DIVMTQSPDSLAVSLGERATINCKSSQSVLFSSNNKNYLSWYQQ KPGQPPKLLIYWASTRESGVPDRFSGSGSGTDFTLTITRLQAED VAVYYCQQYYGLPSTFGQGTKVEIK |
| 103 | Heavy chain variable domain CS2pt1p3_E12K | QVQLQQSGPGLVKPSQTLSLTCVISGDSVSNNDAAWFWIRQSP SRGLEWLGRTHYRSRWYNDYAVSVKSRIRINPDTSKNLFSLHLN SMTPEDSAVYFCARGTPSPKDYSDGGGYYYVEYFQDWGQGTL VTVSS |
| 104 | Light chain variable domain CS2pt1p3_E12K | EIVLTQSPATLSVSPGERATLSCRASQSVSTDLAWYQQKPGQAP RLLIHDASTRATGIPARFTGSGSGTDFTLTISSLEPEDFAVYYCQ QRTNWPLAYTFGRGTKLEIK |
| 105 | Heavy chain variable domain CS3tt1p1_G9K | EVQLVQSGTEVKKPGESLRISCKGSGYTFSNYWIAWVRQRPGK GLEWMGLIYPGDSDTRYSPSIQGQVSLSADKSISTAYLQWTSLK ASDTAVYYCGLGLAAGGRFYYYYHGMDVWGQGTTVIVSS |
| 106 | Light chain variable domain CS3tt1p1_G9K | DIVLTQSPDSLAVSLGETATINCRSSQSVFYSSNNKDYLAWYQH KPGQPPKLLIYWASTRESGVPDRFSGSGSGTDFTLTISSLQAED VAIYYCQQYYTTPQFGQGTKVEIK |
| 107 | Heavy chain variable domain CS3tt1p4_G2K | QVHLVESGGGLVKPGGSLRLSCAASGFTFSDYYMSWIRQTPGK GLEWIAYISSASSYIHYADSVKGRFTVSRDNVHNTLSLLMNGLRV EDTAMYYCARSPMKVLDHWGQGTVVTVSS |
| 108 | Light chain variable domain CS3tt1p4_G2K | EIVMTQSPATLSVSPGERVTLSCRASQSVDNRLAWYQQKPGQP PRLLISGASTRATGIPARFSGSGSGTEFTLTISGLQSEDSAIYYCQ QYNNWPPWTFGQGTKVEI |
| 109 | Heavy chain variable domain CS3tt1p1_A9K | QVQLVESGGGVVQPGRSLRLSCAASGFSFSAYAMHWVRQAPG RGLEWVAVIWFDGSKKVYADSVKGRFTISRDNSKNTLHLQMDSL RAEDTAVYYCSREEYRSFNYWGQGTLVTVS |
| 110 | Light chain variable domain CS3tt1p1_A9K | DIQMTQSPSSLSASVGDRVTIICRASRSIGTNLNWYQQRVGKAP NLLIYDASRLHSGVPSRFSGSGTGTAFTLTISSLQPEDFATYYCQ QSYSTPRTFGPGTKVDSK |
| 111 | Heavy chain variable domain CS2pt1p1_E1K | QVRLQESGPGLIKPSETLSLTCTVSGRSMNDFYWNWIRQPPGK GLEWIGHIYYTGSTTYNPSLESRVVISLDTSKNLFSLQLRSVTAAD TALYYCARQRTPNYFASGTYSNWFDPWGQGIGVTVS |
| 112 | Light chain variable domain CS2pt1p1_E1K | DIVMTQSPDSLAVSLGERATINCKSSQSLLYSANNKNYLAWYQQ KPGQPPKLLIYWASTRESGVPDRFSGSGSGTDFTLTINSLQAAD VAVYSCQQYFATPPTSGQGTKVALK |
| 119 | Heavy chain variable domain CS3pt1p4_C1L | QVQLVQSGAEVKKPGSSVKVSCKASGGTFTDYALSWVRQAPG QGLEWMGGIIPVLGTPHYAQKFEDRVTIIADESTGTVFMALSSLR SDDTGMYYCARGVTHPYYYYAMDVWGQGTTITVSS |
| 120 | Light chain variable domain CS3pt1p4_C1L | QSALTQPASVSGSPGQSISISCTGTSSDVGAYDYVSWYQQHPG KAPRLIIYDVNKRPSGVPYRFSGSKSGTAASLTISGLQSEDEAVY YCGSYTSSSAFFYVFGTGMVTVL |

According to one embodiment of the present invention, the antibody or antigen-binding fragment thereof comprises a heavy chain variable region amino acid sequence of antibody CS2it1p2_F7K (SEQ ID No. 85), or a heavy chain variable region amino acid sequence of antibody CS4tt1p1_E3K (SEQ ID No. 87), or a heavy chain variable region amino acid sequence of antibody CS1pt2p3_E1K (SEQ ID No. 89), or a heavy chain variable region amino acid sequence of antibody CS2pt1p2_E12K (SEQ ID No. 91), or a heavy chain variable region amino acid sequence of antibody CS2pt1p2_A10L (SEQ ID No. 93), or a heavy chain variable region amino acid sequence of antibody CS2tt1p1_C11L (SEQ ID No. 95), or a heavy chain variable region amino acid sequence of antibody CS3tt1p3_A12K (SEQ ID No. 97), or a heavy chain variable region amino acid sequence of antibody CS3pt1p1_E4K (SEQ ID No. 99), or a heavy chain variable region amino acid sequence of antibody CS2tt1p3_C3K (SEQ ID No. 101), or a heavy chain variable region amino acid sequence of antibody CS2pt1p3_E12K (SEQ ID No. 103), or a heavy chain variable region amino acid sequence of antibody CS3tt1p1_G9K (SEQ ID No. 105), or a heavy chain variable region amino acid sequence of antibody CS3tt1p4_G2K (SEQ ID No. 107), or a heavy chain variable region amino acid sequence of antibody CS3tt1p1_A9K (SEQ ID No. 109), or a heavy chain variable region amino acid sequence of antibody CS2pt1p1_E1K (SEQ ID No. 111), or a heavy chain variable region amino acid sequence of antibody CS3pt1p4_C1L (SEQ ID No. 119).

According to an embodiment of the present invention, the antibody or antigen-binding fragment thereof comprises a light chain variable region amino acid sequence of antibody CS2it1p2_F7K (SEQ ID No. 86), or a light chain variable region amino acid sequence of antibody CS4tt1p1_E3K (SEQ ID No. 88), or a light chain variable region amino acid sequence of antibody CS1pt2p3_E1K (SEQ ID No. 90), or a light chain variable region amino acid sequence of antibody CS2pt1p2_E12K (SEQ ID No. 92), or a light chain variable region amino acid sequence of antibody CS2pt1p2_A10L (SEQ ID No. 94), or a light chain variable region amino acid sequence of antibody CS2tt1p1_C11L (SEQ ID No. 96), or a light chain variable region amino acid sequence of antibody CS3tt1p3_A12K (SEQ ID No. 98), or a light chain variable region amino acid sequence of antibody CS3pt1p1_E4K (SEQ ID No. 100), or a light chain variable region amino acid sequence of antibody CS2tt1p3_C3K (SEQ ID No. 102), or a light chain variable region amino acid sequence of antibody CS2pt1p3_E12K (SEQ ID No. 104), or a light chain variable region amino acid sequence of antibody CS3tt1p1_G9K (SEQ ID No. 106), or a light chain variable region amino acid sequence of antibody CS3tt1p4_G2K (SEQ ID No. 108), or a light chain variable region amino acid sequence of antibody CS3tt1p1_A9K (SEQ ID No. 110), or a light chain variable region amino acid sequence of antibody CS2pt1p1_E1K (SEQ ID No. 112), or a light chain variable region amino acid sequence of antibody CS3pt1p4_C1L (SEQ ID No. 120).

According to a preferred embodiment of the present invention, the antibody comprises a heavy chain variable region amino acid sequence of SEQ ID No. 85 and a light chain variable region amino acid sequence of SEQ ID No. 86, or the antibody comprises a heavy chain variable region amino acid sequence of SEQ ID No. 87 and a light chain variable region amino acid sequence of SEQ ID No. 88, or the antibody comprises a heavy chain variable region amino acid sequence of SEQ ID No. 89 and a light chain variable region amino acid sequence of SEQ ID No. 90, or the antibody comprises a heavy chain variable region amino acid sequence of SEQ ID No. 91 and a light chain variable region amino acid sequence of SEQ ID No. 92, or the antibody comprises a heavy chain variable region amino acid sequence of SEQ ID No. 93 and a light chain variable region amino acid sequence of SEQ ID No. 94, or the antibody comprises a heavy chain variable region amino acid sequence of SEQ ID No. 95 and a light chain variable region amino acid sequence of SEQ ID No. 96, or the antibody comprises a heavy chain variable region amino acid sequence of SEQ ID No. 97 and a light chain variable region amino acid sequence of SEQ ID No. 98, or the antibody comprises a heavy chain variable region amino acid sequence of SEQ ID No. 99 and a light chain variable region amino acid sequence of SEQ ID No. 100, or the antibody comprises a heavy chain variable region amino acid sequence of SEQ ID No. 101 and a light chain variable region amino acid sequence of SEQ ID No. 102, or the antibody comprises a heavy chain variable region amino acid sequence of SEQ ID No. 103 and a light chain variable region amino acid sequence of SEQ ID No. 104, or the antibody comprises a heavy chain variable region amino acid sequence of SEQ ID No. 105 and a light chain variable region amino acid sequence of SEQ ID No. 106, or the antibody comprises a heavy chain variable region amino acid sequence of SEQ ID No. 107 and a light chain variable region amino acid sequence of SEQ ID No. 108, or the antibody comprises a heavy chain variable region amino acid sequence of SEQ ID No. 109 and a light chain variable region amino acid sequence of SEQ ID No. 110, or the antibody comprises a heavy chain variable region amino acid sequence of SEQ ID No. 111 and a light chain variable region amino acid sequence of SEQ ID No. 112, or the antibody comprises a heavy chain variable region amino acid sequence of SEQ ID No. 119 and a light chain variable region amino acid sequence of SEQ ID No. 120.

According to a preferred embodiment of the present invention, the antibody used as a source for sequences comprised in the antibody or antigen-binding fragment thereof according to the present invention is selected from the group comprising CS2it1p2_F7K, CS4tt1p1_E3K, CS1pt2p3_E1K, CS2pt1p2_E12K, CS2pt1p2_A10L, CS2tt1p1_C11L, CS3tt1p3_A12K, and CS3pt1p1_E4K, preferably of one antibody from the group comprising CS2it1p2_F7K, CS4tt1p1_E3K, CS1pt2p3_E1K, CS2pt1p2_E12K, CS2pt1p2_A10L, and CS2tt1p1_C11L, more preferably of one antibody from the group comprising CS2it1p2_F7K, CS4tt1p1_E3K, CS1pt2p3_E1K, and CS2pt1p2_E12K, particularly preferably of the antibody CS2it1p2_F7K.

In another embodiment of the present invention, the antibody used as a source for sequences comprised in the antibody of the invention is CS2it1p2_F7K. In another embodiment of the present invention, the antibody used as a source for sequences comprised in the antibody of the invention is CS4tt1p1_E3K. In one embodiment of the present invention, the antibody used as a source for sequences comprised in the antibody of the invention is CS1pt2p3_E1K. In another embodiment of the present invention, the antibody used as a source for sequences comprised in the antibody of the invention is CS2pt1p2_E12K. In another embodiment of the present invention, the antibody used as a source for sequences comprised in the antibody of the invention is CS2pt1p2_A10L. In another embodiment of the present invention, the antibody used as a source for sequences comprised in the antibody of the invention is CS2tt1p1_C11L. In one embodiment of the present invention, the antibody used as a source for sequences comprised in the antibody of the invention is CS3tt1p3_A12K. In another embodiment of the present invention, the antibody used as a source for sequences comprised in the antibody of the invention is CS3pt1p1_E4K. In another embodiment of the present invention, the antibody used as a source for sequences comprised in the antibody of the invention is CS2tt1p3_C3K. In another embodiment of the present invention, the antibody used as a source for sequences comprised in the antibody of the invention is CS2pt1p3_E12K. In one embodiment of the present invention, the antibody used as a source for sequences comprised in the antibody of the invention is CS3tt1p1_G9K. In another embodiment of the present invention, the antibody used as a source for sequences comprised in the antibody of the invention is CS3tt1p4_G2K. In one embodiment of the present invention, the antibody used as a source for sequences comprised in the antibody of the invention is CS3tt1p1_A9K. In another embodiment of the present invention, the antibody used as a source for sequences comprised in the antibody of the invention is CS2pt1p1_E1K.. In another embodiment of the present invention, the antibody used as a source for sequences comprised in the antibody of the invention is CS3pt1p4_C1L

One important achievement of the present invention lies in the identification of novel monoclonal antibodies which are specifically binding to target proteins or epitopes which have not previously been known as potential targets for antibodies directed against HCMV. As confirmed by competition experiments with relevant HCMV antibodies of the prior art, these antibodies apparently bind to novel targets (cf. Figure 5) and are thus able to complement and additively improve neutralization strength of any known antibody of the prior art binding to different targets.

According to the second aspect of the present invention, an antibody or antigen-binding fragment is provided that specifically binds to the gO glycoprotein of *Human Cytomegalovirus,* preferably wherein the antibody or antigen-binding fragment thereof competes with an antibody comprising the combination of the variable region heavy chain amino acid sequence of SEQ ID No. 85 and of the variable region light chain amino acid sequence of SEQ ID No. 86 of antibody CS2it1p2_F7K for binding to *Human Cytomegalovirus.*

Using the sufficient and complete disclosure according to the present invention of the CDR sequences as well as the heavy and light chain variable region sequences of these antibodies, it is straightforward for a skilled person to unambiguously determine the exact composition of the specific epitopes and interacting amino acids in the target structure. In accordance with this, it is made possible by the present invention to provide additional monoclonal antibodies binding to these novel epitopes by using the common general knowledge and well-established technologies available to a person skilled in the art.

Additional monoclonal antibodies binding to these epitopes made available by the present invention can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al. 1988; Hammerling, et al., 1981). In this regard, the term "*monoclonal antibody*" refers to an antibody that is derived from a single clone, including any eukaryotic, prokaryotic, or phage clone, not being restricted to any method by which it is produced.

Based on the recognition of a novel epitope, various methods may be used for generating monoclonal antibodies binding to said epitope. One exemplary method may comprise culturing a hybridoma cell secreting an antibody wherein, preferably, the hybridoma is generated by fusing splenocytes isolated from an animal, e.g., a rat or a mouse, immunized with said novel epitope to which an antibody according to the invention binds, with myeloma cells and then screening the hybridomas resulting from the fusion for hybridoma clones that secrete an antibody able to bind to said epitope.

After immunization of an animal with such a novel epitope to which an antibody of the invention binds, antibodies and/or antibody-producing cells may be obtained from the animal by bleeding or sacrificing the animal. For example, the rat spleen is harvested and splenocytes isolated. The splenocytes are then fused by well-known techniques to any suitable myeloma cells, for example, cells from cell line SP20 available from the American Type Culture Collection (ATCC, Manassas, Va., US).

Hybridomas are selected and cloned by limited dilution. The hybridoma clones are then assayed by methods known in the art for cells that secrete antibodies capable of binding to any such novel epitopes to which the inventive antibodies bind. Ascites fluid, which generally contains high levels of antibodies, can be generated by immunizing rats with positive hybridoma clones.

In another embodiment, antibody-producing immortalized hybridomas may be prepared from the immunized animal. After immunization, the animal is sacrificed and the splenic B cells are fused to immortalized myeloma cells as is well known in the art. See, e.g., Harlow and Lane, supra. In a preferred embodiment, the myeloma cells do not secrete immunoglobulin polypeptides (a non-secretory cell line). After fusion and antibiotic selection, the hybridomas are screened using the novel epitope of interest.

In a preferred embodiment, the initial screening is performed using an enzyme-linked immunosorbent assay (ELISA) or a radioimmunoassay (RIA), preferably an ELISA. An example of ELISA screening is provided in PCT Publication No. WO 00/37504.

Antibody-producing hybridomas which produce antibodies binding to the epitope of interest are selected, cloned, and further screened for desirable characteristics, including robust hybridoma growth, high antibody production, and desirable antibody characteristics. Hybridomas may be cultured and expanded in vivo in syngeneic animals, in animals that lack an immune system, e.g., nude mice, or in cell culture in vitro. Methods of selecting, cloning and expanding hybridomas are well known to those of ordinary skill in the art.

Furthermore, antibodies of the present invention which bind to the same epitope as the antibodies described herein can also be generated using various phage display methods known in the art. In phage display methods, functional antibody domains are displayed on the surface of phage particles which carry the polynucleotide sequences encoding them. Such phage can be utilized to display antigen-binding domains expressed from a repertoire or combinatorial antibody library (e.g., human or murine).

Phages expressing an antigen-binding domain that binds the antigen of interest can be selected or identified with antigen, e.g., using labeled antigen or antigen bound or captured to a solid surface or bead. Phage used in these methods are typically filamentous phage including fd, f1, and M13 binding domains expressed from phage with Fab, Fv, or disulfide stabilized Fv antibody domains recombinantly fused to either the phage gene III or gene VIII protein.

Examples of phage display methods that can be used to make the antibodies of the present invention include those disclosed in Brinkmann et al., 1995; Ames et al., 1995; Kettleborough et al., 1994; Persic et al., 1997; Burton et al., 1994; PCT Publication No. WO 92/01047; PCT Publication Nos. WO 90/02809; WO 91/10737; WO 92/01047; WO 92/18619; WO 93/11236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743; and 5,969,108.

As described in the above references, after phage selection, the antibody coding regions from the phage can be isolated and used to generate whole antibodies including human antibodies or any other desired antigen binding fragment, and expressed in any desired host, including mammalian cells, insect cells, plant cells, yeast, and bacteria, e.g., as described in detail below. For example, techniques to recombinantly produce Fab, Fab', and F(ab')2 fragments can also be employed using methods known in the art such as those disclosed in PCT publication No. WO 92/22324; Mullinax et al., 1992; Sawai et al., 1995; and Better et al. 1988. Examples of techniques which can be used to produce single-chain Fvs and antibodies include those described in U.S. Pat. Nos. 4,946,778 and 5,258,498; Huston et al., 1991; Shu et al., 1993; and Skerra et al., 1988.

Thus, according to a third aspect of the present invention, an antibody or antigen-binding fragment thereof is provided that competes with an antibody comprising the combination of the variable region heavy chain amino acid sequence of SEQ ID No. 87 and of the variable region light chain amino acid sequence of SEQ ID No. 88 of antibody CS4tt1p1_E3K for binding to *Human Cytomegalovirus,* or
an antibody or antigen-binding fragment thereof that competes with an antibody comprising the combination of the variable region heavy chain amino acid sequence of SEQ ID No. 89 and of the variable region light chain amino acid sequence of SEQ ID No. 90 of antibody CS1pt2p3_E1K for binding to *Human Cytomegalovirus,* or
an antibody or antigen-binding fragment thereof that competes with an antibody comprising the combination of the variable region heavy chain amino acid sequence of SEQ ID No. 91 and of the variable region light chain amino acid sequence of SEQ ID No. 92 of antibody CS2pt1p2_E12K for binding to *Human Cytomegalovirus,* or
an antibody or antigen-binding fragment thereof that competes with an antibody comprising the combination of the variable region heavy chain amino acid sequence of SEQ ID No. 93 and of the variable region light chain amino acid sequence of SEQ ID No. 94 of antibody CS2pt1p2_A10L for binding to *Human Cytomegalovirus,* or
an antibody or antigen-binding fragment thereof that competes with an antibody comprising the combination of the variable region heavy chain amino acid sequence of SEQ ID No. 97 and of the variable region light chain amino acid sequence of SEQ ID No. 98 of antibody CS3tt1p3_A12K for binding to *Human Cytomegalovirus,* or
an antibody or antigen-binding fragment thereof that competes with an antibody comprising the combination of the variable region heavy chain amino acid sequence of SEQ ID No. 99 and of the variable region light chain amino acid sequence of SEQ ID No. 100 of antibody CS3pt1p1_E4K for binding to *Human Cytomegalovirus,*
or an antibody or antigen-binding fragment thereof that competes with an antibody comprising the combination of the variable region heavy chain amino acid sequence of SEQ ID No. 101 and of the variable region light chain amino acid sequence of SEQ ID No. 102 of antibody CS2tt1p3_C3K for binding to *Human Cytomegalovirus,*
or an antibody or antigen-binding fragment thereof that competes with an antibody comprising the combination of the variable region heavy chain amino acid sequence of SEQ ID No. 103 and of the variable region light chain amino acid sequence of SEQ ID No. 104 of antibody CS2pt1p3_E12K for binding to *Human Cytomegalovirus,* or
an antibody or antigen-binding fragment thereof that competes with an antibody comprising the combination of the variable region heavy chain amino acid sequence of SEQ ID No. 105 and of the variable region light chain amino acid sequence of SEQ ID No. 106 of antibody CS3tt1p1_G9K for binding to *Human Cytomegalovirus,* or
an antibody or antigen-binding fragment thereof that competes with an antibody comprising the combination of the variable region heavy chain amino acid sequence of SEQ ID No. 109 and of the variable region light chain amino acid sequence of SEQ ID No. 110 of antibody CS3tt1p1_A9K for binding to *Human Cytomegalovirus,* or
an antibody or antigen-binding fragment thereof that competes with an antibody comprising the combination of the variable region heavy chain amino acid sequence of SEQ ID No. 111 and of the variable region light chain amino acid sequence of SEQ ID No. 112 of antibody CS2pt1p1_E1K for binding to *Human Cytomegalovirus.*

In a preferred embodiment of the present invention, the amino acid sequences of the CDRs or of the variable regions comprised therein are from an antibody which is able to neutralize *Human Cytomegalovirus* at a lower IC₅₀ than reference antibody MCMV5322A.

In a preferred embodiment of the present invention, the amino acid sequences of the CDRs or of the variable regions comprised therein are from an antibody which is able to neutralize *Human Cytomegalovirus* at a lower IC₅₀ than reference antibody 4I22.

According to the present invention, the assay(s) for determining IC₅₀ values is/are to be carried out as described in the section "*Virus neutralization tests*" below.

In a preferred embodiment, neutralization potency as IC₅₀ is determined in cells infected with any one of HCMV strains TB40/E or VHL/E. In one preferred embodiment, neutralization potency as IC₅₀ is determined in any one of cell lines HFF-1, ARPE-19 or HEC-LTT.

As already explained above, different subunits of the HCMV are predominantly responsible for infection of specific cell types. Thus, different antibodies may lead to a more pronounced inhibition of infection of specific cell types than other antibodies, depending on their specific binding partner or epitope within the virus.

Accordingly, in one preferred embodiment of the present invention, an antibody or antigen-binding fragment thereof is provided, wherein the neutralization potency in epithelial cells, in particular in HFF-1 cells, has an IC₅₀ of at most 150 ng/ml, preferably at most 100 ng/ml, more preferably at most 50 ng/ml.

Accordingly, in a preferred embodiment of the present invention, an antibody or antigen-binding fragment thereof is provided, wherein the neutralization potency in epithelial cells, in particular in ARPE-19 cells, has an IC₅₀ of less than 50 ng/ml, preferably at most 30 ng/ml, more preferably at most 20 ng/ml.

Accordingly, in a preferred embodiment of the present invention, an antibody or antigen-binding fragment thereof is provided, wherein the neutralization potency in endothelial cells, in particular HEC-LTT cells, has an IC50 of less than 40 ng/ml, preferably at most 30 ng/ml, more preferably at most 20 ng/ml.

In the description of the present application, antibody designations may be used. It is pointed out that the antibodies consist of heavy and light chains which also form part of the present description. If reference is made to an antibody by its designation or to a SEQ ID NO., it should be understood that these ways of reference are interchangeable.

The present invention further relates to a pharmaceutical composition comprising an antibody or antigen-binding fragment thereof according to the invention as defined and further described herein and at least one pharmaceutically acceptable excipient. In one embodiment, the pharmaceutical composition is a vaccination composition for a human and/or animal subject.

The present invention also encompasses a kit comprising an antibody or antigen-binding fragment thereof according to the invention as defined and further described herein and a container.

In one aspect, the present invention is also directed to the antibody or antigen-binding fragment thereof according to the invention as defined and further described herein, the pharmaceutical composition as described herein and the kit for use as a medicament, preferably for use as a vaccine.

In another aspect, the present invention is also directed to the antibody or antigen-binding fragment thereof according to the invention as defined and further described herein, the pharmaceutical composition as described herein and the kit for use in the treatment or prevention of an infection with *Human Cytomegalovirus* in mammalian subjects, preferably in human subjects.

In another aspect, the present invention is directed to a method of treating or preventing an infection with *Human Cytomegalovirus* in mammalian subjects, preferably in human subjects, comprising administering a therapeutically effective amount of at least one antibody and/or antigen-binding fragment thereof as described herein to said subject.

In one aspect of the invention, an antibody and/or antigen-binding fragment thereof according to the invention is administered to a patient in need thereof by intravenous injection or infusion, subcutaneous injection, intramuscular injection, or inhalative application, preferably by intravenous injection.

A pharmaceutical composition of the invention is formulated to be compatible with its intended route of administration. Examples of routes of administration include, but are not limited to, parenteral, e.g., intravenous, intradermal, subcutaneous, oral, intranasal (e.g., inhalation and inhaled through the mouth), transdermal (e.g., topical), transmucosal, and rectal administration.

In a specific embodiment, the composition is formulated in accordance with routine procedures as a pharmaceutical composition adapted for intravenous, subcutaneous, intramuscular, oral, intranasal, or topical administration to human beings. Typically, compositions for intravenous administration are solutions in sterile isotonic aqueous buffer. Where necessary, the composition may also include a solubilizing agent and a local anesthetic such as lignocaine to ease pain at the site of the injection.

The methods of the invention may comprise pulmonary administration, e.g., by use of an inhaler or nebulizer, of a composition formulated with an aerosolizing agent. See, e.g., U.S. Pat. Nos. 6,019,968, 5,985,320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entireties.

The methods of the invention may also comprise administration of a composition formulated for parenteral administration by injection (e.g., by bolus injection or continuous infusion). The pharmaceutical formulation of the present invention may be provided in liquid form or may be provided in lyophilized form.

In one aspect, the present invention relates to a nucleic acid encoding an antibody or antigen-binding fragment thereof as described herein. In another aspect, the present invention relates to an expression vector comprising the nucleic acid as described herein in functional association with an expression control sequence.

In another aspect, the present invention relates to a host cell comprising a nucleic acid as described herein. In one aspect, the present invention relates to a host cell comprising an expression vector as described herein.

In another aspect, the present invention relates to a method of production of an antibody or antigen-binding fragment as described herein, comprising (a) cultivating a host cell as described herein under conditions allowing expression of the antibody or antigen-binding fragment thereof, and (b) recovering the antibody or antigen-binding fragment thereof.

In another embodiment, the present invention relates to an antibody or antigen-binding fragment thereof against *Human Cytomegalovirus* as described herein for use in medicine in combination with at least one further antibody directed against *Human Cytomegalovirus,* wherein said further antibody has a different binding specificity.

In another aspect, the present invention is also directed to the use of the antibody or antigen-binding fragment thereof according to the invention or a pharmaceutical composition of the invention in the manufacture of a medicament for treatment or prevention of an infection with *Human Cytomegalovirus* in mammalian subjects, preferably in human subjects.

All embodiments of the present invention as described and/or claimed herein are deemed to be combinable within the present invention in any combination, unless the skilled person considers such a combination to not make any technical sense or to be excluded by contradiction.

### Examples

### HCMV seropositive individuals and sample collection

Out of the identified cohort of Elite donors (Identification of Elite Neutralizers With Broad and Potent Neutralizing Activity Against Human Cytomegalovirus (HCMV) in a Population of HCMV-Seropositive Blood Donors; Falk et al. 2018) four donors were recruited and PBMCs were isolated under a study protocol approved by the Institutional Review Board of the University of Cologne (study protocol 16-054). HCMV-seropositive blood donors were provided by the German Red Cross Blood-Transfusion Service, Baden-Württemberg and Hessen, with informed consent according to human experimentation guidelines.

### Isolation of peripheral blood mononuclear cells (PBMCs), plasma and total IgG from whole blood.

For the blood draw, collection into EDTA-containing tubes was performed. To isolate PBMCs, Leucosep centrifuge tubes (Greiner Bio-one) prefilled with density gradient separation medium (Histopaque; Sigma-Aldrich) were used according to the manufacturer's instructions. Plasma was collected and stored separately. For IgG isolation, 1 ml of the collected plasma was heat-inactivated (56°C for 40 min) and incubated with Protein G Sepharose (GE Life Sciences) overnight at 4°C. The suspension was transferred to chromatography columns and washed with PBS. IgGs were eluted from Protein G using 0.1 M glycine (pH=3.0) and buffered in 0.1 M Tris (pH=8.0). The buffer exchange to PBS was performed with 30 kDa Amicon spin membranes (Millipore) and the purified IgG concentration was measured using a Nanodrop One Spectrophotometer (A280). The samples were stored at 4°C until use for further experiments.

### HCMV glycoprotein expression and purification.

For B cell isolation and characterization, the Pentamer was purchased from "The NativeAntigen Company", aliquoted and stored by -80°C. Trimer and gH/gL are not commercially available and were generated using replication-defective adenoviral vectors encoding for TR gH-His, gL, plus gO (first described in Siddiquey et al. Jvirol 2021; doi: 10.1128/JVI.02207-20) to infect retinal epithelial cells (ARPE-19). After 24h, the inocula were replaced with medium containing 2% FBS. After seven days, the protein containing supernatants were collected, prefiltered using DEAE resin and purified with Ni-NTA columns (HisTrap HP Cytiva) followed by size exclusion chromatography in PBS (Superdex 200 10/300 GL). The proteins were concentrated over 100kDa filters, flash frozen and stored at -80°C.

### Cell-surface expression of UL_{128/130/131A}-specific antibodies

Variable regions of the antibodies 4I22, 15D8, 8I21, and 8J16 were cloned into a vector containing the heavy and light chain constant regions (pMX-plasmid; Gieselmann et al., 2021. For control, a construct with an EBOV-specific antibody was used (mAb100; Corti et al., 2016. The heavy and light chains were separated by a self-splicing 2A signal sequence and the constant region of the heavy chain fused with a platelet-derived growth factor receptor (PDGFR) transmembrane domain. HEK293T cells were transfected by CaCl₂ precipitation with the antibody construct, harvested two days post-transfection and resuspended in PBS with 2% FBS (Merk) and 2mM EDTA. For analysis, cells were stained by 4',6-Diamidino-2-phenylindole (DAPI; Thermo Fisher Scientific), anti-hulgG-APC (BD), and the Pentamer protein labeled with DyLight 488 (Microscale Antibody Kit, Thermo Fisher Scientific) and analyzed by flow cytometry (Becton Dickinson). Successfully transfected cells were identified by mCherry encoded by the used pMX vectors.

### Isolation of HCMV-specific and whole repertoire IgG⁺ B cells.

B cells were isolated from PBMCs using CD19-microbeads (Miltenyi Biotec) according to the manufacturer's instructions. To identify HCMV-specific B cells, pentameric or trimeric complexes were coupled with fluorescent dyes and used to stain B cells combined with 4',6-Diamidin-2-phenylindol (DAPI; Thermo Fisher Scientific), anti-human CD20-Alexa Fluor 700 (BD), anti-human IgG-APC (BD) and anti-human CD27-PE (BD).

DAPI⁻, CD20⁺, IgG⁺ and bait protein positive cells were sorted using a FACSAria Fusion (Becton Dickinson) in a single-cell manner into 96-well plates. All wells contained 4 µl buffer, consisting of 0.5x PBS, 0.5 U/µl RNAsin (Promega), 0.5 U/µl RNaseOUT (Thermo Fisher Scientific), and 10 mM DTT (Thermo Fisher Scientific). Plates with sorted cells were stored at -80°C until further processing. For whole repertoire sequencing, enriched CD19+ cells were stained with AF700-CD20 (BD), APC-IgG (BD) and DAPI (Thermo Fisher Scientific), and 159,540 to 240,000 DAPI-CD20+IgG+ cells were sorted into FBS containing tubes per donor. After sorting, cells were collected by centrifugation for 5 minutes at 400 × g, supernatant was removed and cell pellets were frozen at -80°C.

### B cell receptor heavy and light chain amplification and sequence analysis.

Single-cell amplification of the heavy and light chains of the B cell receptors was performed as previously described (Gieselmann et al., 2021; *supra*). Briefly, reverse transcription was done with Random Hexamers (Invitrogen), and Superscript IV (Thermo Fisher Scientific). RNA degradation was blocked by the presence of RNaseOUT (Thermo Fisher Sicentific) and RNasin (Promega). The cDNA was generated, and heavy and light chains were amplified using PlatinumTaq HotStart polymerase (Thermo Fisher Scientific) with 6% KB extender combined with an optimized V-gene-specific primer mixes⁶⁶ in a sequential semi-nested approach. PCR products were validated by gel electrophoresis for correct sizes and subjected to Sanger sequencing. For sequence analysis base calls with a Phred score <16 were masked and only sequences with a mean Phred score of 28, less than 15 masked nucleotides and a length of at least 240 nucleotides (nt) were included. Filtered sequences were annotated with IgBLAST (Ye et al., 2013) and trimmed to the variable region from FWR1 to the end of the J-gene.

For determination of clonality all heavy chain sequences were grouped by their VH-gene usage and the pairwise Levenshtein distance for their CDRH3s was determined. B cell receptor sequences with identical VH-gene and a CDRH3 amino acid identity of at least 75% was defined as clonal. All clones were verified taking shared mutations and, whenever available, paired light chain information into account. All B cell sequences were included in the analysis of CDRH3 length and V-gene germline identity distributions. V-gene usage was analyzed with collapsed clonal sequences including only one member of each clone.

### Cloning and in vitro production of monoclonal antibodies.

In vitro generation and production of antibodies from selected B cells was performed as previously described (Gieselmann et al., 2021; *supra*) with minor modifications. In short, variable parts of heavy and light chain were amplified by PCR with extended 2^{nd} PCR primers covering the endogenous leader sequences of all V-genes. PCR fragments were integrated into the corresponding backbone vectors (IgH, IgK, or IgL) by sequence and ligation-independent cloning (SLIC) and sequence controlled plasmids transfected into HEK293-6E cells for expression. Antibodies were finally purified by Protein G-beads.

### Immortalization of B cells.

Peripheral blood was collected from elite donors, which were selected at a prior step. IgG⁺ memory B cells were isolated by binding to CD19 microbeads (Miltenyi) followed by depletion of cells carrying IgM, IgD and IgA by cell sorting. Memory B cells were seeded at 50 cells per well in 96 U-bottom microplates in RPMI medium containing 2.5 µg/ml CpG 2006, 50 U/ml interleucin IL-2, 100 U/ml IL-4, 50 U/ml IL-6 and 25 ng/ml IL-21 in the presence of EBV (30% supernatant of B95-8 cells) and irradiated allogeneic mononuclear cells (20000 per well). After a few weeks positive growing cultures were preceded by single-cell sorting and further analyzed.

### ELISA for determination of antibody binding activities.

For determination of binding activities 96-well ELISA plates (Corning) were coated with 3 µg/ml target protein in PBS at 4°C overnight, washed 3x with PBST (PBS, 0.05% Tween-20) and blocked with PBST / 5% Nonfat dried milk powder (Panreac AppliChem) for 60 min at RT. For virus interaction assays, supernatant of HCMV TB40/E-infected HFF cells was used instead of target protein. Antibodies were tested at 5-fold dilutions with starting concentrations of 30 µg/ml for monoclonal antibodies and 500 µg/ml for plgG. For gH/gL depletion assays 0,6 mg/ml plgG were preincubated with 0,04 mg/ml gH/gL Dimer protein (15-fold plgG excess) at 4°C overnight to block antibodies targeting gH/gL epitopes and the resulting mixtures were used for ELISA analysis afterwards.

The samples of interest were incubated for 90 min at RT, plates washed again, and incubated with horseradish peroxidase-conjugated goat anti-human IgG antibody (Jackson ImmunoResearch, 1:2000 in PBST / 5% Nonfat dried milk powder) for 45 min at RT. Development was done by 2,2'-azino-bis(3-ethylbenzothiazoline-6-sulphonic acid) solution (ABTS, Thermo Fisher Scientific) and substrate measured at an absorbance (γ= 415 nm - 695 nm) with absorbance reader (Tecan) after fixed incubation times. All curves were verified manually and positive binding was defined by an increasing absorbance with a final signal of at least 0.2 (Trimer and Dimer) or 0.1 (Pentamer) and an EC50 < 150 µg/ml.

### Epitope mapping by antibody-competition ELISA.

For antibody competition experiments 96-well ELISA plates (Corning) were coated with 3 µg/ml Pentamer in PBS at 4°C overnight, washed three times with PBST (PBS, 0.05% Tween-20) and blocked with PBST / 5% Nonfat dried milk powder (Panreac AppliChem) for 60 min at RT. Antibodies with unknown epitopes (first antibodies) were incubated at concentration of 30 µg/ml with target protein for 90 min at RT, plates washed again, and biotin-labeled reference antibodies (second antibodies) were added for 45 min at RT. The required concentration of biotinylated second antibodies was taken at the EC50 level.

Then, plates were incubated with horseradish peroxidase-conjugated Streptavidin (Jackson ImmunoResearch, 1 :5000 in PBST / 5% Nonfat dried milk powder) for 30 min at RT. Development was done by 2,2'-azino-bis(3-ethylbenzothiazoline-6-sulphonic acid) solution (ABTS, Thermo Fisher Scientific) and substrate measured at an absorbance (γ= 415 nm - 695 nm) with absorbance reader (Tecan) after fixed incubation times. Positive competition was defined by a signal reduction of 40% compared to signal without first antibody competitor. Clustering of antibodies with a similar competition pattern was performed using Morpheus Cluster (software.broadinstitute.org/morpheus/).

### Receptor binding and receptor competition assay.

For receptor interaction analysis NRP2 (Sino Biological), PDGFRα (Sino Biological) and TGFβR3 (Sino Biological) in PBS were coated to 96-well ELISA plates (Corning) with 5 µg/ml, 3 µg/ml, and 3 µg/ml, respectively. After incubation at 4°C overnight, plates were washed 3x with PBST (PBS, 0.05% Tween-20) and blocked with PBST / 5% Nonfat dried milk powder (Panreac AppliChem) for 60 min at RT. Biotin-labeled Trimer, Dimer or Pentamer constructs were added at 5-fold dilutions with starting concentrations of 10 µg/ml for PDGFRα and TGFβR3 or 100 µg/ml for NRP2 and incubated for 90 min at RT.

For block of receptor interaction by isolated antibodies, biotinylated Trimer or Pentamer at EC50 concentration were preincubated with 30 µg/ml of investigated antibody for 60 min at RT before addition to coated respective cellular receptors. Detection of receptor-protein interaction was measured by staining with Streptavidin-HRP (Jackson ImmunoResearch, 1 :5000 in PBST / 5% Nonfat dried milk powder) as described for antibody competition experiments. Positive competition was defined by a signal reduction of 40% compared to signal without antibody competitor. Results of competition experiments are shown in Figure 5.

### Virus neutralization tests.

HFFs, ARPE 19 or HEC-LTTs were seeded in 96-well plates at 1.5 × 10⁴ cells per well. Antibody samples were serially diluted, mixed with TB40/E or other virus stock (VHL/E; Towne; TR; AD169; Toledo; VR18; Merlin) at an infection multiplicity (MOI) of 1 infectious unit per cell or below, and preincubated for 2 hours at 37 °C. Subsequently, the virus-antibody mixtures were added to the target cell and incubated overnight at 37 °C. The cells were then fixed with 80% acetone (Sigma Aldrich) and stained with a monoclonal antibody against HCMV immediate early antigen [E13, (Argene Biosoft), CH160 (Abcam), or clone63-27 (Plachter B et al.. Analysis of proteins encoded by IE regions 1 and 2 of human cytomegalovirus using monoclonal antibodies generated against recombinant antigens. Virology. 1993 Apr;193(2):642-52. doi: 10.1006/viro.1993.1172. PMID: 7681609.)] and a Cy3-conjugated secondary antibody (Goat Anti-Mouse IgG (H+L), Jackson ImmunoResearch).

Infection rates were measured and calculated by ImageXpress Pico and the corresponding dose-response curves were analyzed by non-linear regression to determine the antibody concentration at which the infection rate is reduced to 50 % of the maximum. This concentration is given as the half-maximal neutralization concentration (IC₅₀). To analyze the effect of antibodies on clinical isolates, the procedure was modified as follows. Recent clinical isolates grow strictly cell associated, thus preventing direct use for neutralization assays.

To release cell-free infectivity, fibroblast cultures, in which such isolates were growing, were treated with siRNAs against the viral genes RL13 and UL128 as previously described (Weiler et al., 2022). Cell-free supernatants were then harvested after 6 days and used for neutralization assays. Infection multiplicities reached with these supernatants were always below 1 infectious unit per cell. The first letter of an isolate indicates the material, from which the virus was grown (here: T = throat washing). The second letter indicates the underlying condition (here: P = pediatric; H = hematologic). The third letter and number are a pseudonym of the patient.

### Determination of synergistic scores.

To analyze, whether antibodies act additive in neutralization of HCMV, dilution series of either antibody were prepared, and all possible combinations were tested for their neutralizing effect. Dose response curves were analyzed with the online tool "synergyfinder" (https://synergyfinder.org) using a matrix data format, and synergy scores were calculated using the Zero Interaction Potency (ZIP) model. According to the recommendation in the user guide, the interaction was regarded likely to be additive if the score was between -10 and 10 and synergistic or antagonistic if the score was higher or lower.

### Quantification of Adsorption and Penetration of HCMV.

In order to investigate how our antibodies of the elite neutralizers affect the adsorption or penetration of HCMV, the purified IgGs were screened using a dual-fluorescent HCMV strain TB40-BACKL7-UL32EGFP-UL100mCherry as previously described (Laib Sampaio et al., 2021) and non-neutralizing HCMV-negative IgG was used as a negative control. HFFs were seeded on gelatin-coated 8-well µ-slide (lbidi) at a density of 4 × 10^4 cells/well and fresh virus was preincubated with IgG samples at concentrations of at least 3 times the NT50 for 2 hours at 37°C to ensure a significant reduction of infectivity.

The mixtures of virus and IgG were then added to HFFs and incubated for additional 2 hours, washed with medium and incubated for additional 20 min at 37 °C. The cells were then fixed with 4% paraformaldehyde and permeabilized with PBS containing 1% fetal bovine serum (PAN Biotech), 10% sucrose (Sigma-Aldrich) and 0.5% Nonidet P40 (Sigma-Aldrich), and nuclei were then stained with DAPI (Sigma-Aldrich). Pictures of each condition were taken using a fluorescence microscope and evaluated for green and red dot-like fluorescence signals. Combined green and red fluorescence signals indicate intact enveloped virions that are attached to cells, while isolated green fluorescence without red indicates virions that have penetrated cells. In each experiment, twenty cells were randomly taken for each condition, and the numbers of particles with green and red fluorescence or green fluorescence without red were counted. Each setup was independently repeated at least five times.

### Pharmacokinetic profile assessment

To investigate monoclonal antibody *in vivo* pharmacokinetic profiles, 0.5 mg of antibody in DPBS was intravenously injected into *B6.Cg-Fcgr^{tm1Dcr} Prkdc^{scid}* Tg(FCGRT)32Dcr/ DcrJ mice (The Jackson Laboratory) that transgenically express the human neonatal Fc receptor and carry a murine *Fcgrt* gene knock-out. In addition to HCMV neutralizing antibodies, the HIV-1 antibodies 3BNC117 and 10-1074 in clinical investigation were included as controls (n=4 per group; Scheid et al., 2011; Mouquet et al., 2012; Mendoza et al., 2018).

Serum was collected from longitudinally acquired blood samples by centrifugation at 10,000 g for 5 min at room temperature (RT) and stored at -20°C until the determination of human serum IgG levels by ELISA. To this end, high-binding ELISA plates (Corning) were coated overnight with goat anti-human IgG (Jackson Immunoresearch). After blocking with 2% bovine serum albumin (Carl Roth), 1 µM EDTA (Thermo Fisher), and 0.1% Tween-20 (Carl Roth) in PBS (blocking buffer), a 1:3 dilution series of each serum sample in PBS (starting at 1:20) was added and incubated at RT for 90 min.

Subsequently, HRP-conjugated goat anti-human IgG (Jackson Immunoresearch) in blocking buffer was added and incubated for at least 90 min. After addition of ABTS (Thermo Fisher), optical density was determined using a microplate reader (Tecan). Between each step, the plate was washed with 0.05% Tween-20 in PBS. Human serum IgG levels were calculated based on plate-specific antibody standard curves determined by duplicate dilution series of the SARS-CoV-2 neutralizing monoclonal human antibody R568-1G9 included on each plate (Vanshylla et al., 2022).

### Generation of Fab fragments from IgG

IgG at 1 mg/ml in PBS (CS2it1p2_F7K, CS4tt1p1_E3K, CS3pt1p4_C1L, or CS2pt1p2_A10L) was incubated overnight at 37°C with 1:2000 (wt/wt) Lys-C Endoproteinase (ThermoFisher). To inactivate the Lys-C, 1X complete EDTA-free protease inhibitor cocktail (Roche) was added to each reaction. Fabs were then purified using CaptureSelect IgG-CH1 affinity resin according to the manufacturer's instructions (ThermoFisher)

### Quantification and statistical analysis

Flow cytometry analysis and quantifications were done by FlowJo10. Quantitative and statistical analyses were performed using GraphPad Prism (v9), Microsoft Excel for Mac (v16.44), and Python (v3.6.8). Sequence alignment was done by IgBlast, MacVector, Clustal Omega 1.2.3, and Geneious. Microscopic analysis were done by ImageXpress Pico. For structural analysis cryoSPARC, Phenix, COOT, ISOLDE, and ChimeraX were used.

### References

1. EI Helou, Guy, and Raymund R. Razonable. "Safety considerations with current and emerging antiviral therapies for cytomegalovirus infection in transplantation." Expert opinion on drug safety 18.11 (2019): 1017-1030.
2. Sandonis, Virginia, et al. "Role of neutralizing antibodies in CMV infection: implications for new therapeutic approaches." Trends in microbiology 28.11 (2020): 900-912.
3. Gerna, Giuseppe, et al. "Monoclonal antibodies to different components of the human cytomegalovirus (HCMV) pentamer gH/gL/pUL128L and trimer gH/gL/gO as well as antibodies elicited during primary HCMV infection prevent epithelial cell syncytium formation." Journal of virology 90.14 (2016): 6216-6223.
4. Zhou, Momei, Jean-Marc Lanchy, and Brent J. Ryckman. "Human cytomegalovirus gH/gL/gO promotes the fusion step of entry into all cell types, whereas gH/gL/UL128-131 broadens virus tropism through a distinct mechanism." Journal of virology 89.17 (2015): 8999-9009.
5. Ishida, Julie H., et al. "Phase 2 randomized, double-blind, placebo-controlled trial of RG7667, a combination monoclonal antibody, for prevention of cytomegalovirus infection in high-risk kidney transplant recipients." Antimicrobial agents and chemotherapy 61.2 (2017): e01794-16.
6. Deng, Rong, et al. "Pharmacokinetics and exposure-response analysis of RG7667, a combination of two anticytomegalovirus monoclonal antibodies, in a phase 2a randomized trial to prevent cytomegalovirus infection in high-risk kidney transplant recipients." Antimicrobial agents and chemotherapy 62.2 (2018): e01108-17.
7. Macagno, Annalisa, et al. "Isolation of human monoclonal antibodies that potently neutralize human cytomegalovirus infection by targeting different epitopes on the gH/gL/UL128-131A complex." Journal of virology 84.2 (2010): 1005-1013.
8. III, C. R., et al. "Design and construction of a hybrid immunoglobulin domain with properties of both heavy and light chain variable regions." Protein Engineering, Design and Selection, Volume 10, Issue 8, (1997): 949-957
9. Holliger, Philipp, and Peter J. Hudson. "Engineered antibody fragments and the rise of single domains." Nature biotechnology 23.9 (2005): 1126-1136.
10. Lonberg, Nils. "Human antibodies from transgenic animals." Nature biotechnology 23.9 (2005): 1117-1125.
11. Jefferis, Roy, and Marie-Paule Lefranc. "Human immunoglobulin allotypes: possible implications for immunogenicity." MAbs. Vol. 1. No. 4. Taylor & Francis, 2009.
12. Ausubel, F. M. "In FM Ausubel et al. eds." Current protocols in molecular biology. Greene Publication Associates and Wiley-Interscience, New York (1987).
13. Angal, S., et al. "A single amino acid substitution abolishes the heterogeneity of chimeric mouse/human (IgG4) antibody." Molecular immunology 30.1 (1993): 105-108.
14. Kabat, E. A., et al. "Sequences of Proteins of Immunological Interest, 5th edit." National Institutes of Health, Bethesda, MD (1991).
15. Chothia, Cyrus, and Arthur M. Lesk. "Canonical structures for the hypervariable regions of immunoglobulins." Journal of molecular biology 196.4 (1987): 901-917.
16. Lefranc, Marie-Paule, et al. "IMGT, the international ImMunoGeneTics database." Nucleic acids research 27.1 (1999): 209-212.
17. Harlow, E. D., and David Lane. "A laboratory manual." New York: Cold Spring Harbor Laboratory 579 (1988): 44.
18. Hammerling, G. J., et al. "In Monoclonal Antibodies and T-Cell Hybridomas." Research Monographs in Immunology No. 3 (1981).
19. Brinkmann, Ulrich, et al. "Phage display of disulfide-stabilized Fv fragments." Journal of Immunological Methods 182.1 (1995): 41-50.
20. Ames, Robert S., et al. "Conversion of murine Fabs isolated from a combinatorial phage display library to full length immunoglobulins." Journal of Immunological Methods 184.2 (1995): 177-186.
21. Kettleborough, Catherine A., et al. "Isolation of tumor cell-specific single-chain Fv from immunized mice using phage-antibody libraries and the re-construction of whole antibodies from these antibody fragments." European journal of immunology 24.4 (1994): 952-958.
22. Persic, Lidija, et al. "An integrated vector system for the eukaryotic expression of antibodies or their fragments after selection from phage display libraries." Gene 187.1 (1997): 9-18.
23. Burton, Dennis R., and Carlos F. Barbas III. "Human antibodies from combinatorial libraries." Advances in immunology 57 (1994): 191-280.
24. Mullinax, R. L., et al. "Expression of a heterodimeric Fab antibody protein in one cloning step." Biotechniques 12.6 (1992): 864-869.
25. Sawai, Hideaki, et al. "Direct production of the fab fragment derived from the sperm immobilizing antibody using polymerase chain reaction and cDNA expression vectors." American Journal of reproductive immunology 34.1 (1995): 26-34.
26. Better, Marc, et al. "Escherichia coli secretion of an active chimeric antibody fragment." Science 240.4855 (1988): 1041-1043.
27. Huston, James S., et al. "[3] Protein engineering of single-chain Fv analogs and fusion proteins." Methods in enzymology. Vol. 203. Academic Press, 1991. 46-88.
28. Shu, Liming, et al. "Secretion of a single-gene-encoded immunoglobulin from myeloma cells." Proceedings of the National Academy of Sciences 90.17 (1993): 7995-7999.
29. Skerra, Arne, and Andreas Plückthun. "Assembly of a functional immunoglobulin Fv fragment in Escherichia coli." Science 240.4855 (1988): 1038-1041.
30. Gieselmann, Lutz, et al. "Effective high-throughput isolation of fully human antibodies targeting infectious pathogens." Nature Protocols 16.7 (2021): 3639-3671.
31. Corti, Davide, et al. "Protective monotherapy against lethal Ebola virus infection by a potently neutralizing antibody." Science 351.6279 (2016): 1339-1342.
32. Ye, Jian, et al. "IgBLAST: an immunoglobulin variable domain sequence analysis tool." Nucleic acids research 41.W1 (2013): W34-W40.
33. Weiler, Nina, et al. "Combined knockdown of RL13 and UL128 for release of cell-free infectivity from recent HCMV isolates." Journal of Virological Methods 305 (2022): 114537.
34. Laib Sampaio, Kerstin, et al. "Selection of Human Cytomegalovirus Mutants with Resistance against PDGFRα-Derived Entry Inhibitors." Viruses 13.6 (2021): 1094.
35. Scheid, Johannes F., et al. "Sequence and structural convergence of broad and potent HIV antibodies that mimic CD4 binding." Science 333.6049 (2011): 1633-1637.
36. Mouquet, Hugo, et al. "Complex-type N-glycan recognition by potent broadly neutralizing HIV antibodies." Proceedings of the National Academy of Sciences 109.47 (2012): E3268-E3277.
37. Mendoza, Pilar, et al. "Combination therapy with anti-HIV-1 antibodies maintains viral suppression." Nature 561.7724 (2018): 479-484.
38. Vanshylla, Kanika, et al. "Discovery of ultrapotent broadly neutralizing antibodies from SARS-CoV-2 elite neutralizers." Cell host & microbe 30.1 (2022): 69-82.

## Claims

1. Antibody or antigen-binding fragment thereof directed against *Human Cytomegalovirus (HCMV; Human Betaherpesvirus 5)*, wherein the antibody or antigen-binding fragment thereof comprises the combination of the heavy chain CDR1 to CDR3 and the light chain CDR1 to CDR3 amino acid sequence of one antibody selected from the group comprising:
**CS2it1p2_F7K** (having a CDR-H1 amino acid sequence of SEQ ID No. 1, a CDR-H2 amino acid sequence of SEQ ID No. 2, a CDR-H3 amino acid sequence of SEQ ID No. 3, a CDR-L1 amino acid sequence of SEQ ID No. 4, a CDR-L2 amino acid sequence of SEQ ID No. 5, a CDR-L3 amino acid sequence of SEQ ID No. 6),
**CS4tt1p1_E3K** (having a CDR-H1 amino acid sequence of SEQ ID No. 7, a CDR-H2 amino acid sequence of SEQ ID No. 8, a CDR-H3 amino acid sequence of SEQ ID No. 9, a CDR-L1 amino acid sequence of SEQ ID No. 10, a CDR-L2 amino acid sequence of SEQ ID No. 11, a CDR-L3 amino acid sequence of SEQ ID No. 12),
**CS1pt2p3_E1K** (having a CDR-H1 amino acid sequence of SEQ ID No. 13, a CDR-H2 amino acid sequence of SEQ ID No. 14, a CDR-H3 amino acid sequence of SEQ ID No. 15, a CDR-L1 amino acid sequence of SEQ ID No. 16, a CDR-L2 amino acid sequence of SEQ ID No. 17, a CDR-L3 amino acid sequence of SEQ ID No. 18),
**CS2pt1p2_E12K** (having a CDR-H1 amino acid sequence of SEQ ID No. 19, a CDR-H2 amino acid sequence of SEQ ID No. 20, a CDR-H3 amino acid sequence of SEQ ID No. 21, a CDR-L1 amino acid sequence of SEQ ID No. 22, a CDR-L2 amino acid sequence of SEQ ID No. 23, a CDR-L3 amino acid sequence of SEQ ID No. 24),
**CS2pt1p2_A10L** (having a CDR-H1 amino acid sequence of SEQ ID No. 25, a CDR-H2 amino acid sequence of SEQ ID No. 26, a CDR-H3 amino acid sequence of SEQ ID No. 27, a CDR-L1 amino acid sequence of SEQ ID No. 28, a CDR-L2 amino acid sequence of SEQ ID No. 29, a CDR-L3 amino acid sequence of SEQ ID No. 30),
**CS2tt1p1_C11L** (having a CDR-H1 amino acid sequence of SEQ ID No. 31, a CDR-H2 amino acid sequence of SEQ ID No. 32, a CDR-H3 amino acid sequence of SEQ ID No. 33, a CDR-L1 amino acid sequence of SEQ ID No. 34, a CDR-L2 amino acid sequence of SEQ ID No. 35, a CDR-L3 amino acid sequence of SEQ ID No. 36),
**CS3tt1p3_A12K** (having a CDR-H1 amino acid sequence of SEQ ID No. 37, a CDR-H2 amino acid sequence of SEQ ID No. 38, a CDR-H3 amino acid sequence of SEQ ID No. 39, a CDR-L1 amino acid sequence of SEQ ID No. 40, a CDR-L2 amino acid sequence of SEQ ID No. 41, a CDR-L3 amino acid sequence of SEQ ID No. 42),
**CS3pt1p1_E4K** (having a CDR-H1 amino acid sequence of SEQ ID No. 43, a CDR-H2 amino acid sequence of SEQ ID No. 44, a CDR-H3 amino acid sequence of SEQ ID No. 45, a CDR-L1 amino acid sequence of SEQ ID No. 46, a CDR-L2 amino acid sequence of SEQ ID No. 47, a CDR-L3 amino acid sequence of SEQ ID No. 48),
**CS2tt1p3_C3K** (having a CDR-H1 amino acid sequence of SEQ ID No. 49, a CDR-H2 amino acid sequence of SEQ ID No. 50, a CDR-H3 amino acid sequence of SEQ ID No. 51, a CDR-L1 amino acid sequence of SEQ ID No. 52, a CDR-L2 amino acid sequence of SEQ ID No. 53, a CDR-L3 amino acid sequence of SEQ ID No. 54),
**CS2pt1p3_E12K** (having a CDR-H1 amino acid sequence of SEQ ID No. 55, a CDR-H2 amino acid sequence of SEQ ID No. 56, a CDR-H3 amino acid sequence of SEQ ID No. 57, a CDR-L1 amino acid sequence of SEQ ID No. 58, a CDR-L2 amino acid sequence of SEQ ID No. 59, a CDR-L3 amino acid sequence of SEQ ID No. 60),
**CS3tt1p1_G9K** (having a CDR-H1 amino acid sequence of SEQ ID No. 61, a CDR-H2 amino acid sequence of SEQ ID No. 62, a CDR-H3 amino acid sequence of SEQ ID No. 63, a CDR-L1 amino acid sequence of SEQ ID No. 64, a CDR-L2 amino acid sequence of SEQ ID No. 65, a CDR-L3 amino acid sequence of SEQ ID No. 66),
**CS3tt1p4_G2K** (having a CDR-H1 amino acid sequence of SEQ ID No. 67, a CDR-H2 amino acid sequence of SEQ ID No. 68, a CDR-H3 amino acid sequence of SEQ ID No. 69, a CDR-L1 amino acid sequence of SEQ ID No. 70, a CDR-L2 amino acid sequence of SEQ ID No. 71, a CDR-L3 amino acid sequence of SEQ ID No. 72),
**CS3tt1p1_A9K** (having a CDR-H1 amino acid sequence of SEQ ID No. 73, a CDR-H2 amino acid sequence of SEQ ID No. 74, a CDR-H3 amino acid sequence of SEQ ID No. 75, a CDR-L1 amino acid sequence of SEQ ID No. 76, a CDR-L2 amino acid sequence of SEQ ID No. 77, a CDR-L3 amino acid sequence of SEQ ID No. 78),
**CS2pt1p1_E1K** (having a CDR-H1 amino acid sequence of SEQ ID No. 79, a CDR-H2 amino acid sequence of SEQ ID No. 80, a CDR-H3 amino acid sequence of SEQ ID No. 81, a CDR-L1 amino acid sequence of SEQ ID No. 82, a CDR-L2 amino acid sequence of SEQ ID No. 83, a CDR-L3 amino acid sequence of SEQ ID No. 84), and
**CS3pt1p4_C1L** (having a CDR-H1 amino acid sequence of SEQ ID No. 113, a CDR-H2 amino acid sequence of SEQ ID No. 114, a CDR-H3 amino acid sequence of SEQ ID No. 115, a CDR-L1 amino acid sequence of SEQ ID No. 116, a CDR-L2 amino acid sequence of SEQ ID No. 117, a CDR-L3 amino acid sequence of SEQ ID No. 118).

2. Antibody or antigen-binding fragment thereof according to claim 1, wherein the antibody or antigen-binding fragment thereof comprises the combination of the variable region heavy chain amino acid sequence and of the variable region light chain amino acid sequence of one antibody selected from the group comprising:
**CS2it1p2_F7K** (having the variable region heavy chain amino acid sequence of SEQ ID No. 85 and the variable region light chain amino acid sequence of SEQ ID No. 86),
**CS4tt1p1_E3K** (having the variable region heavy chain amino acid sequence of SEQ ID No. 87 and the variable region light chain amino acid sequence of SEQ ID No. 88),
**CS1pt2p3_E1K** (having the variable region heavy chain amino acid sequence of SEQ ID No. 89 and the variable region light chain amino acid sequence of SEQ ID No. 90),
**CS2pt1p2_E12K** (having the variable region heavy chain amino acid sequence of SEQ ID No. 91 and the variable region light chain amino acid sequence of SEQ ID No. 92),
**CS2pt1p2_A10L** (having the variable region heavy chain amino acid sequence of SEQ ID No. 93 and the variable region light chain amino acid sequence of SEQ ID No. 94),
**CS2tt1p1_C11L** (having the variable region heavy chain amino acid sequence of SEQ ID No. 95 and the variable region light chain amino acid sequence of SEQ ID No. 96),
**CS3tt1p3_A12K** (having the variable region heavy chain amino acid sequence of SEQ ID No. 97 and the variable region light chain amino acid sequence of SEQ ID No. 98),
**CS3pt1p1_E4K** (having the variable region heavy chain amino acid sequence of SEQ ID No. 99 and the variable region light chain amino acid sequence of SEQ ID No. 100),
**CS2tt1p3_C3K** (having the variable region heavy chain amino acid sequence of SEQ ID No. 101 and the variable region light chain amino acid sequence of SEQ ID No. 102),
**CS2pt1p3_E12K** (having the variable region heavy chain amino acid sequence of SEQ ID No. 103 and the variable region light chain amino acid sequence of SEQ ID No. 104),
**CS3tt1p1_G9K** (having the variable region heavy chain amino acid sequence of SEQ ID No. 105 and the variable region light chain amino acid sequence of SEQ ID No. 106),
**CS3tt1p4_G2K** (having the variable region heavy chain amino acid sequence of SEQ ID No. 107 and the variable region light chain amino acid sequence of SEQ ID No. 108),
**CS3tt1p1_A9K** (having the variable region heavy chain amino acid sequence of SEQ ID No. 109 and the variable region light chain amino acid sequence of SEQ ID No. 110),
**CS2pt1p1_E1K** (having the variable region heavy chain amino acid sequence of SEQ ID No. 111 and the variable region light chain amino acid sequence of SEQ ID No. 112), and
**CS3pt1p4_C1L** (having the variable region heavy chain amino acid sequence of SEQ ID No. 119 and the variable region light chain amino acid sequence of SEQ ID No. 120).

3. Antibody or antigen-binding fragment thereof according to claim 1 or 2, wherein the amino acid sequences comprised are of one antibody selected from the group comprising **CS2it1p2_F7K, CS4tt1p1_E3K, CS1pt2p3_E1K, CS2pt1p2_E12K, CS2pt1p2_A10L, CS2tt1p1_C11L, CS3tt1p3_A12K,** and **CS3pt1p1_E4K,** preferably of one antibody from the group comprising **CS2it1p2_F7K, CS4tt1p1_E3K, CS1pt2p3_E1K, CS2pt1p2_E12K, CS2pt1p2_A10L,** and **CS2tt1p1_C11L,** more preferably of one antibody from the group comprising **CS2it1p2_F7K, CS4tt1p1_E3K, CS1pt2p3_E1K,** and **CS2pt1p2_E12K,** particularly preferably of the antibody **CS2it1p2_F7K.**

4. Antibody or antigen-binding fragment thereof that specifically binds to the gO glycoprotein of *Human Cytomegalovirus,* preferably wherein the antibody or antigen-binding fragment thereof competes with an antibody comprising the combination of the variable region heavy chain amino acid sequence of SEQ ID No. 85 and of the variable region light chain amino acid sequence of SEQ ID No. 86 of antibody **CS2it1p2_F7K** for binding to *Human Cytomegalovirus.*

5. Antibody or antigen-binding fragment thereof that competes with an antibody comprising the combination of the variable region heavy chain amino acid sequence of SEQ ID No. 87 and of the variable region light chain amino acid sequence of SEQ ID No. 88 of antibody **CS4tt1p1_E3K** for binding to *Human Cytomegalovirus,* or
antibody or antigen-binding fragment thereof that competes with an antibody comprising the combination of the variable region heavy chain amino acid sequence of SEQ ID No. 89 and of the variable region light chain amino acid sequence of SEQ ID No. 90 of antibody **CS1pt2p3_E1K** for binding to *Human Cytomegalovirus,* or
antibody or antigen-binding fragment thereof that competes with an antibody comprising the combination of the variable region heavy chain amino acid sequence of SEQ ID No. 91 and of the variable region light chain amino acid sequence of SEQ ID No. 92 of antibody **CS2pt1p2_E12K** for binding to *Human Cytomegalovirus, or*
antibody or antigen-binding fragment thereof that competes with an antibody comprising the combination of the variable region heavy chain amino acid sequence of SEQ ID No. 93 and of the variable region light chain amino acid sequence of SEQ ID No. 94 of antibody **CS2pt1p2_A10L** for binding to *Human Cytomegalovirus,* or
antibody or antigen-binding fragment thereof that competes with an antibody comprising the combination of the variable region heavy chain amino acid sequence of SEQ ID No. 97 and of the variable region light chain amino acid sequence of SEQ ID No. 98 of antibody **CS3tt1p3_A12K** for binding to *Human Cytomegalovirus,* or
antibody or antigen-binding fragment thereof that competes with an antibody comprising the combination of the variable region heavy chain amino acid sequence of SEQ ID No. 99 and of the variable region light chain amino acid sequence of SEQ ID No. 100 of antibody **CS3pt1p1_E4K** for binding to *Human Cytomegalovirus,* or
antibody or antigen-binding fragment thereof that competes with an antibody comprising the combination of the variable region heavy chain amino acid sequence of SEQ ID No. 101 and of the variable region light chain amino acid sequence of SEQ ID No. 102 of antibody **CS2tt1p3_C3K** for binding to *Human Cytomegalovirus,* or
antibody or antigen-binding fragment thereof that competes with an antibody comprising the combination of the variable region heavy chain amino acid sequence of SEQ ID No. 103 and of the variable region light chain amino acid sequence of SEQ ID No. 104 of antibody **CS2pt1p3_E12K** for binding to *Human Cytomegalovirus,* or
antibody or antigen-binding fragment thereof that competes with an antibody comprising the combination of the variable region heavy chain amino acid sequence of SEQ ID No. 105 and of the variable region light chain amino acid sequence of SEQ ID No. 106 of antibody **CS3tt1p1_G9K** for binding to *Human Cytomegalovirus,* or
antibody or antigen-binding fragment thereof that competes with an antibody comprising the combination of the variable region heavy chain amino acid sequence of SEQ ID No. 109 and of the variable region light chain amino acid sequence of SEQ ID No. 110 of antibody **CS3tt1p1_A9K** for binding to *Human Cytomegalovirus,* or
antibody or antigen-binding fragment thereof that competes with an antibody comprising the combination of the variable region heavy chain amino acid sequence of SEQ ID No. 111 and of the variable region light chain amino acid sequence of SEQ ID No. 112 of antibody **CS2pt1p1_E1K** for binding to *Human Cytomegalovirus.*

6. Antibody or antigen-binding fragment thereof according to any one of claims 1 to 5, wherein the amino acid sequences of the CDRs or of the variable regions comprised therein are from an antibody which is able to neutralize *Human Cytomegalovirus* at a lower IC₅₀ than reference antibody MCMV5322A, wherein the amino acid sequences of the CDRs or of the variable regions comprised therein are not from the antibody CS2pt1p2_A10L.

7. Antibody or antigen-binding fragment thereof according to any one of claims 1 to 3 or an antibody or antigen-binding fragment thereof that competes with an antibody comprising the combination of the variable region heavy chain amino acid sequence of SEQ ID No. 93 and of the variable region light chain amino acid sequence of SEQ ID No. 94 of antibody **CS2pt1p2_A10L** for binding to *Human Cytomegalovirus,* wherein the amino acid sequences of the CDRs or of the variable regions comprised therein are from an antibody which is able to neutralize *Human Cytomegalovirus* at a lower IC₅₀ than reference antibody 4I22.

8. Antibody or antigen-binding fragment thereof according to any one of claims 6 or 7, wherein neutralization potency as IC₅₀ is determined in cells infected with any one of HCMV strains TB40/E or VHL/E, and/or wherein neutralization potency as IC₅₀ is determined in any one of cell lines HFF-1, ARPE-19 or HEC-LTT.

9. Antibody or antigen-binding fragment thereof according to any one of claims 1 to 8, wherein the neutralization potency in fibroblasts, in particular in HFF-1 cells, has an IC₅₀ of at most 150 ng/ml, preferably at most 100 ng/ml, more preferably at most 50 ng/ml, and/or wherein the neutralization potency in epithelial cells, in particular in ARPE-19 cells, has an IC₅₀ of less than 50 ng/ml, preferably at most 30 ng/ml, more preferably at most 20 ng/ml, and/or wherein the neutralization potency in endothelial cells, in particular HEC-LTT cells, has an IC₅₀ of less than 40 ng/ml, preferably at most 30 ng/ml, more preferably at most 20 ng/ml.

10. Pharmaceutical composition comprising an antibody or antigen-binding fragment thereof according to any one of claims 1 to 9 and at least one pharmaceutically acceptable excipient.

11. Kit comprising an antibody or antigen-binding fragment thereof according to any one of claims 1 to 9 and a container.

12. Antibody or antigen-binding fragment thereof according to any one of claims 1 to 19, pharmaceutical composition according to claim 10, or kit according to claim 11 for use as a medicament.

13. Antibody or antigen-binding fragment thereof according to any one of claims 1 to 9, pharmaceutical composition according to claim 10, or kit according to claim 11 for use in the treatment or prevention of an infection with *Human Cytomegalovirus* in mammalian subjects, preferably in human subjects.

14. Nucleic acid encoding an antibody or antigen-binding fragment thereof according to any of claims 1 to 9.

15. An expression vector comprising the nucleic acid of claim 14 in functional association with an expression control sequence.

16. Host cell comprising a nucleic acid according to claim 14 or the expression vector according to claim 15.

17. Method of production of an antibody or antigen-binding fragment thereof according to any one of claims 1 to 9, comprising
(a) cultivating the host cell of claim 16 under conditions allowing expression of the antibody or antigen-binding fragment thereof, and
(b) recovering the antibody or antigen-binding fragment thereof.
